# EUROPÄISCHE PATENTSCHRIFT

(11) **EP 2 008 608 B1**
(45) Veröffentlichungstag und Bekanntmachung des Hinweises auf die Patenterteilung: **15.08.2018**
(21) Anmeldenummer: 08157231.5
(22) Anmeldetag: 29.05.2008
(51) Int. Cl.: A61B 50/20

(54) **Chirurgische Halterung für einen chirurgischen Behälter und chirurgischer Behälter**
Surgical mount for a surgical container and surgical container
Fixation chirurgicale pour un récipient chirurgical et récipient chirurgical

(30) Priorität: 25.06.2007 DE 102007030863
(43) Veröffentlichungstag der Anmeldung: 31.12.2008
(60) Teilanmeldung: 11174084.1
(73) Patentinhaber: Aesculap AG, 78532 Tuttlingen (DE)
(72) Erfinder: Thomas, Stefan, 78532 Tuttlingen (DE)
(74) Vertreter: Hoeger, Stellrecht & Partner Patentanwälte mbB

(56) Entgegenhaltungen:
- WO-A-99/44717
- DE-A1- 2 812 712
- DE-U1-202005 015 415
- DE-U1-202007 000 931
- US-A- 5 098 676

## Beschreibung

Die Erfindung betrifft eine chirurgische Halterung für chirurgische Instrumente und/oder Implantate für einen chirurgischen Behälter, insbesondere einen Sterilbehälter oder Siebkorb, umfassend eine Lagereinrichtung zum Halten und/oder Lagern von chirurgischen Instrumenten und/oder Implantaten und eine Befestigungseinrichtung zum Befestigen der Halterung am Behälter, wobei die Lagereinrichtung mindestens ein Lagerelement zum Halten und/oder Lagern von chirurgischen Instrumenten und/oder Implantaten und mindestens einen Halteteil zum Halten des mindestens einen Lagerelements umfasst und dass der mindestens eine Halteteil von einer Haltestellung, in welcher das mindestens eine Lagerelement an dem mindestens einen Halteteil gehalten ist, in eine Entnahmestellung überführbar ist, in welcher das mindestens eine Lagerelement und der mindestens eine Halteteil voneinander lösbar sind.

Ferner betrifft die Erfindung einen chirurgischen Behälter zum Lagern und/oder Sterilisieren und/oder Reinigen von chirurgischen Instrumenten und/oder Implantaten, umfassend einen durch einen Boden und Seitenwände definierten Aufnahmeraum und mindestens eine chirurgische Halterung für chirurgische Instrumente und/oder Implantate.

Mithilfe einer chirurgischen Halterung der eingangs beschriebenen Art kann ein chirurgisches Instrument und/oder ein Implantat in definierter Weise, zum Beispiel an einer bestimmten Position, im Aufnahmeraum eines chirurgischen Behälters gehalten und/oder gelagert werden. Eine Mehrzahl von chirurgischen Instrumenten und/oder Implantaten kann übersichtlich in dem Behälter angeordnet werden. Diese können auch dann an ihrer jeweiligen Position gehalten werden, wenn der Behälter bewegt wird, und sind somit gegen Beschädigungen, beispielsweise Stöße gegeneinander, besser geschützt.

Ein Beispiel für eine derartige chirurgische Halterung ist in dem Gebrauchsmuster DE 20 2005 015 415 U1 beschrieben. Sie umfasst in Form von Einzelteilen ein Profilelement zum Halten und Lagern von Instrumenten sowie Befestigungselemente. Zum Befestigen dieser Halterung an einem Sterilbehälter werden die Befestigungselemente an eine Behälteraußenwand angelegt, wobei an den Befestigungselementen vorgesehene Haltearme durch jeweils eine Durchbrechung des Behälters geführt und in Eingriff mit dem an der Behälterinnenwand anliegenden Profilelement gebracht werden. Nachteilig bei einer derartigen Halterung ist, dass zu ihrer Befestigung am Behälter mehrere Einzelteile gehandhabt werden müssen und dass insbesondere Handgriffe außerhalb und innerhalb des Behälters vorgenommen werden müssen.

Eine chirurgische Halterung sowie ein chirurgischer Behälter der eingangs beschriebenen Art sind beispielsweise aus der DE 28 12 712 A1 sowie der US 6,244,447 B11 bekannt. Weitere chirurgische Behälter werden in der DE 20 2007 000 931 U1, WO 99/44717 A1 sowie US 5,098,676 offenbart.

Daher ist es Aufgabe der vorliegenden Erfindung, eine chirurgische Halterung für einen chirurgischen Behälter und einen chirurgischen Behälter der eingangs beschriebenen Art derart zu verbessern, dass die Halterung am Behälter einfacher befestigt werden kann.

Diese Aufgabe wird erfindungsgemäß dadurch gelöst, dass die Halterung einen Halteteil umfasst und dass der Halteteil und die Befestigungseinrichtung einstückig gearbeitet sind.

Dies ermöglicht eine Vielzahl möglicher Kombinationen zwischen unterschiedlichen Lagerelementen und Halteteilen. Beispielsweise kann ein Halteteil in Abhängigkeit von den zu haltenden Instrumenten und/oder Implantaten gezielt mit einem Lagerelement versehen werden, das zum Halten dieser Instrumente und/oder Implantate geeignet ist. Während das mindestens eine Lagerelement an dem mindestens einen Halteteil in der Haltestellung stabil gehalten werden kann, bietet die Überführbarkeit des mindestens einen Halteteils in die Entnahmestellung die Möglichkeit, die Vorgänge des Verbindens und des Lösens des mindestens einen Lagerelements und des mindestens einen Halteteils häufig zu wiederholen, ohne dass starke Verschleißerscheinungen auftreten. Besonders günstig ist es, dass der mindestens eine Halteteil und die mindestens zwei Befestigungsglieder einstückig gearbeitet sind. Sie können in diesem Fall gemeinsam, beispielsweise in einem Spritz- oder Verformungsschritt, hergestellt werden.

Ferner ist es vorteilhaft, wenn die Befestigungseinrichtung von einer Befestigungsstellung, in welcher sie mit dem Behälter in Eingriff ist und verbunden ist, in eine Anlegestellung überführbar ist, in welcher sie mit dem Behälter außer Eingriff bringbar ist, und wenn die Befestigungseinrichtung mindestens zwei über die Lagereinrichtung miteinander verbundene Befestigungsglieder zum Befestigen der Halterung am Behälter umfasst.

Dadurch, dass die Befestigungsglieder über die Lagereinrichtung miteinander verbunden sind, kann zum Beispiel die Halterung als Ganzes, also insbesondere die Befestigungseinrichtung zusammen mit der daran gehaltenen Lagereinrichtung, innerhalb des Aufnahmeraums eines Behälters von einer Seite einer Seitenwand oder des Bodens aus an die Seitenwand oder den Boden herangeführt und dort befestigt werden. Ein Verbinden mehrerer Einzelteile der Halterung miteinander während ihrer Befestigung am Behälter, beispielsweise nach einem Durchgreifen der Befestigungseinrichtung durch am Behälter vorgesehene Durchbrechungen, ist nicht notwendig. Der Arbeitsschritt der Befestigung der Halterung am Behälter wird vereinfacht und ist somit schneller durchführbar.

Die Lagereinrichtung kann einteilig oder mehrteilig ausgebildet sein. Im Falle einer einteiligen Ausbildung der Lagereinrichtung kann auch die gesamte Halterung einteilig ausgebildet und gegebenenfalls einstückig gearbeitet sein. Bei einer Ausführungsform einer mehrteiligen Lagereinrichtung umfasst diese vorzugsweise mindestens ein Lagerelement zum Haltern von chirurgischen Instrumenten und mindestens einen Halteteil zum Halten des mindestens einen Lagerelements. Dies bietet die Möglichkeit, jeweils unterschiedliche Halteteile und Lagerelemente miteinander zu kombinieren. In diesem Fall sind die Befestigungsglieder vorteilhafterweise über den mindestens einen Halteteil miteinander verbunden. Auf einfache Weise können dann beispielsweise zunächst das mindestens eine Lagerelement an dem mindestens einen Halteteil und anschließend die vollständige aus ihren Einzelteilen zusammengesetzte Halterung am Behälter befestigt werden.

Durch die Überführbarkeit der Befestigungseinrichtung von der Befestigungsstellung in die Anlegestellung wird es ermöglicht, das Befestigen der Halterung am Behälter und das Abnehmen der Halterung vom Behälter häufig zu wiederholen, ohne dass starke Verschleißerscheinungen auftreten. Beispielsweise kann ein Behälter, der eine Vielzahl von zur Befestigung der Halterung geeigneten Positionen aufweist, je nach Anzahl und Art der zu lagernden Instrumente und/oder Implantate flexibel mit unterschiedlich ausgeformten Halterungen an unterschiedlichen Positionen versehen werden.

Durch die Überführbarkeit der Befestigungseinrichtung von der Befestigungsstellung in die Anlegestellung wird es ermöglicht, das Befestigen der Halterung am Behälter und das Abnehmen der Halterung vom Behälter häufig zu wiederholen, ohne dass starke Verschleißerscheinungen auftreten. Beispielsweise kann ein Behälter, der eine Vielzahl von zur Befestigung der Halterung geeigneten Positionen aufweist, je nach Anzahl und Art der zu lagernden Instrumente und/oder Implantate flexibel mit unterschiedlich ausgeformten Halterungen an unterschiedlichen Positionen versehen werden.

Günstig ist es, dass eine Rückstelleinrichtung zum Überführen der Befestigungseinrichtung von der Anlegestellung in die Befestigungsstellung vorgesehen ist. Wenn während des Befestigens der Befestigungseinrichtung am Behälter die Rückstelleinrichtung betätigt wird, kann dadurch sichergestellt werden, dass die Befestigungseinrichtung die Befestigungsstellung einnimmt und somit am Behälter stabil befestigt ist und bleibt.

Vorteilhafterweise ist mindestens eines der mindestens zwei Befestigungsglieder relativ zu der Lagereinrichtung und/oder zu einem anderen Befestigungsglied beweglich angeordnet. In diesem Fall kann die Überführung der Befestigungseinrichtung von der Befestigungsstellung in die Anlegestellung durch eine Bewegung dieses Befestigungsgliedes relativ zu der Lagereinrichtung und/oder zu einem anderen Befestigungsglied durchgeführt werden.

Günstigerweise stehen die Befestigungsglieder von der Lagereinrichtung ab. Dadurch sind sie auf einfache Weise an den Boden oder eine Seitenwand des Behälters führbar, und der Befestigungsvorgang wird durch eine gegebenenfalls voluminöse Ausformung der Lagereinrichtung so wenig gestört wie möglich.

Günstig ist es, wenn die Rückstelleinrichtung mindestens ein Rückstellelement zum Überführen der Befestigungseinrichtung von der Anlegestellung in die Befestigungsstellung umfasst, welches einem der mindestens zwei Befestigungsglieder zugeordnet ist. In diesem Fall kann jedes Rückstellelement genutzt werden, um jeweils das Befestigungsglied, dem es zugeordnet ist, in die Stellung zu überführen, die dieses einnimmt, wenn die gesamte Befestigungseinrichtung die Befestigungsstellung einnimmt.

Vorzugsweise ist das mindestens eine Rückstellelement in Form eines elastisch federnden Abschnitts des Befestigungsgliedes ausgebildet. Damit kann die Befestigungsstellung insbesondere eine Grundstellung der Befestigungseinrichtung definieren. Die Überführung in die Anlegestellung kann zum Beispiel dadurch erreicht werden, dass mindestens ein Befestigungsglied entgegen der Federkraft seines elastisch federnden Abschnitts ausgelenkt wird. Die auslenkende Kraft muss temporär aufgebracht werden, um die Befestigungseinrichtung in der Anlegestellung zu halten. In der Anlegestellung ist sie an den Behälter anlegbar. Wenn die auslenkende Kraft nach dem Anlegen an den Behälter zurückgenommen wird, zwingt die Federkraft des elastisch federnden Abschnitts das mindestens eine Befestigungsglied in die Grundstellung und somit auch die Befestigungsstellung zurück. Ohne ein erneutes Auslenken des mindestens einen Befestigungsgliedes ist somit kein Ablösen der Befestigungseinrichtung vom Behälter möglich.

Vorteilhafterweise ist ein Abstand zwischen den mindestens zwei Befestigungsgliedern in der Anlegestellung größer oder kleiner als in der Befestigungsstellung. Dadurch kann die Befestigungseinrichtung durch eine einfache Bewegung der Befestigungsglieder relativ zueinander in die Anlegestellung überführt werden.

Besonders vorteilhaft ist es, wenn der Abstand zwischen freien Enden der mindestens zwei Befestigungsglieder definiert ist. Wenn die Befestigungsglieder beispielsweise Rückstellelemente in Form elastisch federnder Abschnitte aufweisen und durch eine Auslenkung der Befestigungsglieder gegen die Federkraft des elastisch federnden Abschnitts in die Anlegestellung überführbar sind, ist der zwischen ihren freien Enden definierte Abstand durch eine derartige Auslenkung relativ stark vergrößerbar oder verkleinerbar.

Vorzugsweise umfasst mindestens eines der mindestens zwei Befestigungsglieder mindestens ein Kupplungselement, wobei das mindestens eine Kupplungselement eine auf die Lagereinrichtung hin oder im Wesentlichen hin weisende erste Kupplungsoberfläche zum Anlegen an eine korrespondierende Befestigungsfläche eines chirurgischen Behälters aufweist, und wobei die Halterung mindestens eine von der Lagereinrichtung weg oder im Wesentlichen weg weisende zweite Kupplungsoberfläche zum Anlegen an mindestens eine, vorzugsweise korrespondierende, Befestigungsfläche eines chirurgischen Behälters aufweist. Wenn die erste und die mindestens eine zweite Kupplungsoberfläche in der Befestigungsstellung jeweils an einer, vorzugsweise korrespondierenden, Befestigungsfläche anliegen, kann die Halterung nicht durch Druck oder Zug, der in der Richtung einer Verbindungslinie zwischen der Lagereinrichtung und dem Kupplungselement ausgeübt wird, bewegt werden. Wenn die ersten und zweiten Kupplungsoberflächen beispielsweise parallel zu einem Behälterboden verlaufen, kann die Halterung durch einen senkrecht zum Boden ausgeübten Zug nicht von dem Behälter abgenommen werden.

Insbesondere ist es vorteilhaft, wenn die erste Kupplungsoberfläche eine erste Kupplungsebene definiert und die zweite Kupplungsoberfläche eine zweite Kupplungsebene definiert, wobei die erste und die zweite Kupplungsebene parallel oder im Wesentlichen parallel zueinander sind und wobei die erste Kupplungsebene von der Lagereinrichtung weiter beabstandet ist als die zweite Kupplungsebene. Kupplungselemente mit einer derartigen räumlichen Anordnung der Kupplungsoberflächen können beispielsweise so mit Stegen in Eingriff gebracht werden, die zwischen an dem Behälter vorgesehenen Durchbrechungen liegen, dass die erste und die zweite Kupplungsoberfläche eines Kupplungselements an voneinander weg weisenden Oberflächen eines Stegs anliegen.

Gemäß einer bevorzugten Ausführungsform der Erfindung kann vorgesehen sein, dass das mindestens eine Kupplungselement in Form einer Ausnehmung mit einer ersten Seitenfläche ausgebildet ist und dass die erste Seitenfläche die erste Kupplungsoberfläche definiert. Das Vorsehen einer Ausnehmung an einem Befestigungsglied stellt eine einfache Methode zur Erzeugung eines Kupplungselementes dar.

Bei einer weiteren bevorzugten Ausführungsform der Erfindung kann vorgesehen sein, dass das mindestens eine Kupplungselement in Form eines Vorsprungs mit einer ersten Seitenfläche ausgebildet ist und dass die erste Seitenfläche die erste Kupplungsoberfläche definiert. Auch ein derartiger Vorsprung kann auf einfache Weise an einem Befestigungsglied ausgebildet werden.

Bei einer weiteren bevorzugten Ausführungsform der Erfindung kann vorgesehen sein, dass das mindestens eine Kupplungselement in Form eines umgebogenen freien Endes des mindestens einen Befestigungsglieds ausgebildet ist, wobei das umgebogene freie Ende eine erste Seitenfläche aufweist und die erste Seitenfläche die erste Kupplungsoberfläche definiert. Auch das mindestens eine Kupplungselement dieser Ausführungsform ist auf einfache Weise herstellbar.

Vorteilhafterweise kann die mindestens eine zweite Kupplungsoberfläche an dem mindestens einen Kupplungselement vorgesehen sein. Dadurch können beispielsweise ein oder mehrere Kupplungselemente jeweils eine erste und eine zweite Kupplungsoberfläche aufweisen. Die Lagereinrichtung muss dann keine weitere zum Anlegen an eine Befestigungsfläche eines Behälters geeignete Oberfläche aufweisen. Sie kann zum Beispiel nach dem Befestigen der Halterung an einem Boden eines Behälters von dem Boden beabstandet bleiben, so dass chirurgische Instrumente und/oder Implantate, aber auch weitere Objekte wie beispielsweise Kabel, Platinen oder Kleinmotoren, zwischen der Lagereinrichtung und dem Behälterboden gelagert und/oder gehalten, beispielsweise eingeklemmt, werden können.

Vorteilhafterweise ist das mindestens eine Kupplungselement in Form einer Ausnehmung, eines Vorsprungs oder eines umgebogenen freien Endes des mindestens einen Befestigungsglieds ausgebildet und weist eine zweite Seitenfläche auf, wobei die zweite Seitenfläche die mindestens eine zweite Kupplungsoberfläche definiert. Bei der Herstellung derartiger Kupplungselemente kann auf einfache Weise eine für die Befestigung der Halterung am Behälter vorteilhafte gegenseitige Positionierung der ersten und der zweiten Seitenfläche erreicht werden.

Es kann auch Vorteile bieten, wenn die mindestens eine zweite Kupplungsoberfläche an der Lagereinrichtung vorgesehen ist. Die Halterung kann an einem Behälter besonders stabil befestigt sein, wenn die Lagereinrichtung direkt am Behälter anliegt.

Günstig ist es, wenn die Befestigungseinrichtung mindestens eine Anlegefläche aufweist, die sich senkrecht oder im Wesentlichen senkrecht zu der ersten und/oder der zweiten Kupplungsoberfläche erstreckt und die in der Befestigungsstellung an eine, vorzugsweise korrespondierende, Befestigungsfläche des Behälters anlegbar ist. Dies erschwert eine Bewegung der am Behälter befestigten Befestigungseinrichtung in einer auf diese, vorzugsweise korrespondierende, Befestigungsfläche hin weisenden Richtung. Durch das Vorhandensein von zwei voneinander weg oder im Wesentlichen weg weisenden Anlegeflächen an der Befestigungseinrichtung kann die Halterung in der Befestigungsstellung zum Beispiel zwischen den beiden entsprechenden korrespondierenden Befestigungsflächen des Behälters so stabil gelagert werden, dass jedes Verrücken entlang der Verbindungslinie zwischen den beiden Befestigungsflächen stark erschwert wird.

Vorteilhaft ist es, wenn die mindestens eine Anlegefläche an mindestens einem Befestigungsglied vorgesehen ist. Da die Befestigungseinrichtung mit Hilfe der Befestigungsglieder am Behälter befestigt wird, dient es der Einfachheit der Konstruktion, wenn auch die mindestens eine Anlegefläche hier vorgesehen ist. Wenn an dem mindestens einen Befestigungsglied beispielsweise ein Kupplungselement in Form einer Ausnehmung vorgesehen ist, kann eine Grundfläche der Ausnehmung die Anlegefläche definieren.

Bei einer bevorzugten Ausführungsform der Erfindung kann vorgesehen sein, dass die Lagereinrichtung mindestens ein Lagerelement zum Halten und/oder Lagern von chirurgischen Instrumenten und/oder Implantaten und mindestens einen Halteteil zum Halten des mindestens einen Lagerelements umfasst und dass die Befestigungseinrichtung mindestens zwei über den mindestens einen Halteteil miteinander verbundene Befestigungsglieder umfasst. Dadurch wird beispielsweise ein Austauschen des mindestens einen Lagerelements ermöglicht. Lagerelemente können beispielsweise unterschiedlich ausgeformt werden, um jeweils zum Halten bestimmter chirurgischer Instrumente und/oder Implantate besser geeignet zu sein. Dann können ein oder mehrere Halteteile einer oder mehrerer Halterungen in Abhängigkeit von den zu haltenden Instrumenten und/oder Implantaten mit einem oder mehreren entsprechenden Lagerelementen versehen werden. Bei Beschädigungen an dem mindestens einen Halteteil oder an dem mindestens einen Lagerelement muss nur das jeweils betroffene Einzelteil ausgewechselt werden, nicht die gesamte Halterung. Dadurch, dass die Lagereinrichtung mehrere Einzelteile umfasst, wird es weiterhin ermöglicht, jeden der Teile aus einem der Funktion des jeweiligen Teils angemessenen Werkstoff herzustellen. So kann beispielsweise das mindestens eine Lagerelement aus einem Kunststoff gefertigt sein, wodurch weniger Kratzer und Schleifspuren an den an ihm gelagerten chirurgischen Instrumenten und/oder Implantaten auftreten, der mindestens eine Halteteil dagegen beispielsweise aus einem Metall.

Günstig ist es, wenn der mindestens eine Halteteil zwischen den mindestens zwei Befestigungsgliedern angeordnet ist. Ein zwischen zwei jeweils an einem Behälter befestigbaren Befestigungsgliedern angeordneter Halteteil kann besonders stabil an seiner Position gehalten werden.

Vorteilhaft ist es, wenn mindestens eines der mindestens zwei Befestigungsglieder von dem mindestens einen Halteteil um einen Gliedwinkel abgewinkelt ist. Dadurch wird das Heranführen der Befestigungsglieder an einen Boden oder eine Seitenwand eines Behälters erleichtert.

Die Halterung kann einfach an einen Boden oder eine Seitenwand eines Behälters herangeführt werden, wenn der Gliedwinkel circa 20° bis circa 160° beträgt. Besonders günstig ist es, wenn der Gliedwinkel circa 80° bis circa 150° beträgt.

Bei einer bevorzugten Ausführungsform der Erfindung ist der mindestens eine Halteteil in Form eines ebenen oder im Wesentlichen ebenen Stegs ausgebildet. Dadurch wird beispielsweise eine einfache Herstellung eines mit zwei Befestigungsgliedern einstückig gearbeiteten Halteteils ermöglicht. Dabei werden von einem mittleren Abschnitt eines flachen Streifens, beispielsweise aus einem elastischen Metallwerkstoff, zwei dem mittleren Abschnitt in einer Längsrichtung des Streifens benachbarte äußere Abschnitte des Streifens abgewinkelt. Der mittlere Abschnitt definiert den Halteteil, die äußeren Abschnitte zwei Befestigungsglieder einer erfindungsgemäßen Halterung.

Günstig ist es, wenn an dem mindestens einen Lagerelement mindestens eine Halteteilaufnahme vorgesehen ist und der mindestens eine Halteteil mit der mindestens der einen Halteteilaufnahme in Eingriff bringbar ist. Beispielsweise kann jeweils ein Halteteil mit einer Halteteilaufnahme form- und/oder kraftschlüssig in Eingriff gebracht werden, so dass das mindestens eine Lagerelement an dem mindestens einen Halteteil stabil gehalten ist.

Bei einer bevorzugten Ausführungsform der Erfindung ist die mindestens eine Halteteilaufnahme in Form einer Halteteildurchbrechung des Lagerelements ausgebildet und der mindestens eine Halteteil durch die mindestens eine Halteteildurchbrechung führbar. Eine Halteteildurchbrechung kann an dem Lagerelement auf besonders einfache Weise ausgebildet werden. Wenn die Halteteildurchbrechung sich beispielsweise zwischen zwei Seiten des Lagerelements erstreckt und ein durch die Halteteildurchbrechung geführter Halteteil auf den beiden Seiten des Lagerelements jeweils mit mindestens einem Befestigungsglied verbunden ist, können das Lagerelement und der Halteteil nicht voneinander getrennt werden, solange die Halterung über die Befestigungsglieder an einem Behälter befestigt ist. Somit ist das Lagerelement stabil an dem Halteteil gehalten. Ein beispielsweise in Form eines ebenen oder im Wesentlichen ebenen Stegs ausgebildetes Halteteil kann auf einfache Weise durch eine an dem Lagerelement vorgesehene Halteteildurchbrechung geführt werden.

Um die Stabilität der Halterung zu erhöhen, kann vorgesehen sein, dass mindestens zwei Halteteile über mindestens ein Stabilisierungselement miteinander verbunden sind. Beispielsweise können durch die Verbindung von zwei stegförmigen Halteteilen über ein ebenfalls stegförmiges Stabilisierungselement in Abhängigkeit von den Positionen, an denen das Stabilisierungselement mit den Halteteilen verbunden ist, verschieden geformte Bauteile, zum Beispiel solche mit H- oder U-förmigen Strukturen, erhalten werden.

Ferner kann die Stabilität der Halterung auch dadurch erhöht werden, dass mindestens zwei mit unterschiedlichen Halteteilen verbundene Befestigungsglieder über mindestens ein Stabilisierungselement miteinander verbunden sind. Dadurch können die relativen Positionen dieser Halteteile zueinander festgelegt werden, ohne dass die Halteteile direkt miteinander verbunden werden. Sie können beispielsweise mit verschiedenen Halteteilaufnahmen eines Lagerelements in Eingriff gebracht werden. Ein Lagerelement mit mehreren Halteteilaufnahmen kann flexibel entweder an mehreren, voneinander getrennten Bauteilen gehalten werden, die jeweils ein Halteteil und mit dem jeweiligen Halteteil verbundene Befestigungsglieder umfassen, oder an nur einem Bauteil, in dem mindestens ein Stabilisierungselement mit unterschiedlichen Halteteilen verbundene Befestigungsglieder miteinander verbindet.

Optional kann auch bei einer Halterung der eingangs beschriebenen Art vorgesehen sein, dass die Lagereinrichtung mindestens ein Lagerelement zum Halten und/oder Lagern von chirurgischen Instrumenten und/oder Implantaten und mindestens einen Halteteil zum Halten des mindestens einen Lagerelements umfasst und dass der mindestens eine Halteteil von einer Haltestellung, in welcher das mindestens eine Lagerelement an dem mindestens einen Halteteil gehalten ist, in eine Entnahmestellung überführbar ist, in welcher das mindestens eine Lagerelement und der mindestens eine Halteteil voneinander lösbar sind.

Vorzugsweise ist die Befestigungseinrichtung von einer Befestigungsstellung, in welcher sie mit dem Behälter in Eingriff bringbar und verbindbar ist, in eine Anlegestellung überführbar, in welcher sie mit dem Behälter außer Eingriff bringbar ist. Dadurch kann die Befestigungseinrichtung auf einfache Weise am Behälter befestigt werden.

Insbesondere umfasst die Befestigungseinrichtung vorzugsweise mindestens zwei über den mindestens einen Halteteil der Lagereinrichtung miteinander verbundene Befestigungsglieder. Der mindestens eine Halteteil kann dann zusammen mit den Befestigungsgliedern und gegebenenfalls dem mindestens einen Lagerelement innerhalb des Aufnahmeraums eines Behälters an den Boden oder eine Seitenwand herangeführt und dort befestigt werden, ohne dass dabei ein Verbinden mehrerer Einzelteile der Halterung miteinander notwendig ist.

Vorzugsweise umfasst der mindestens eine Halteteil mindestens ein Halteglied zum Halten des mindestens einen Lagerelements in der Haltestellung. Ein derartiges Halteglied kann zum Beispiel so ausgebildet sein, dass das mindestens eine Lagerelement mit ihm in der Haltestellung des Halteteils in Eingriff gebracht werden kann.

Insbesondere umfasst der mindestens eine Halteteil bevorzugt mindestens zwei Halteglieder. Ein Lagerelement kann stabiler an einem Halteteil gehalten werden, wenn es beispielsweise an zwei oder drei Haltegliedern des Halteteils gehalten wird. Ein Lagerelement kann auch zwischen zwei oder drei Haltegliedern in der Haltestellung einklemmbar sein.

Günstig ist es, wenn die mindestens zwei Halteglieder relativ zueinander beweglich angeordnet sind. So kann zum Beispiel die Überführung eines Halteteils von der Haltestellung in die Entnahmestellung durch eine Bewegung von mindestens zwei Haltegliedern des Halteteils relativ zueinander durchgeführt werden. Beispielsweise kann ein Lagerelement in der Entnahmestellung zwischen zwei Halteglieder eines Halteteils eingeführt werden. Bei der anschließenden Überführung des Halteteils von der Entnahmestellung in die Haltestellung verringert sich ein Abstand der Halteglieder zueinander. In der Haltestellung können die Halteglieder schließlich mit dem Lagerelement in Eingriff sein oder das Lagerelement einklemmen.

Vorteilhafterweise sind die mindestens zwei Halteglieder relativ zueinander verschwenkbar angeordnet. Auch im Fall fest miteinander verbundener Halteglieder kann die Beweglichkeit der Halteglieder relativ zueinander durch Verschwenkbarkeit auf einfache Weise sichergestellt werden.

Günstig ist es, wenn die mindestens zwei Halteglieder jeweils ein erstes Ende und ein zweites Ende aufweisen und an ihren ersten Enden über eine Gelenkverbindung miteinander verbunden sind. Dadurch können die Halteglieder über die gesamte Erstreckung zwischen ihrem ersten Ende und ihrem zweiten Ende relativ zueinander verschwenkt werden. In der Entnahmestellung kann ein Lagerelement an eine sich vom ersten bis zum zweiten Ende eines Haltegliedes erstreckende Oberfläche dieses Haltegliedes angenähert und in der Haltestellung an dieser gehalten werden.

Vorzugsweise ist die Gelenkverbindung in Form eines einfachen Scharniers, eines Filmscharniers oder eines Federscharniers ausgebildet. Derartige Scharniere sind technisch umfassend optimierte Beispiele für Gelenkverbindungen.

Bei einer bevorzugten Ausführungsform der Erfindung kann ein Befestigungsglied die Gelenkverbindung umfassen. Dadurch wird die Konstruktion der Halterung stark vereinfacht. Wenn ein Befestigungsglied beispielsweise aus einem elastisch biegbaren Metallblech hergestellt und mit zwei Haltegliedern verbunden ist, können die Halteglieder relativ zueinander unter gleichzeitigem Verbiegen des Metallblechs des Befestigungsgliedes bewegt werden. Weiterhin kann bei dieser Ausgestaltung ein Befestigungsglied an den ersten Enden der Halteglieder vorgesehen sein und somit für eine Befestigung an einem Behälter vorteilhaft von der Lagereinrichtung abstehen.

Vorteilhafterweise ist an dem zweiten Ende mindestens eines Haltegliedes ein Befestigungsglied angeordnet. Auch an dieser Position kann ein Befestigungsglied exponiert angeordnet werden. Wenn ein Halteglied beispielsweise an seinem ersten und an seinem zweiten Ende mit jeweils einem Befestigungsglied verbunden ist, können die beiden Befestigungsglieder beim Befestigen der Halterung an einem Behälter unabhängig von einer gegebenenfalls voluminösen Ausformung der Lagereinrichtung einfach mit jeweils einer Hand an den Behälter geführt und dort befestigt werden.

Günstigerweise weist das mindestens eine Halteglied mindestens ein Halteelement zum form- und/oder kraftschlüssigen Halten des mindestens einen Lagerelements in der Haltestellung auf. Dadurch wird die Stabilität der Halterung des mindestens einen Lagerelements am Halteteil erhöht.

Vorzugsweise weist mindestens ein Halteelement mindestens eines Halteglieds auf ein anderes Halteglied hin oder im Wesentlichen hin. Dies ermöglicht ein stabileres Halten eines Lagerelements, das von mindestens zwei Haltegliedern gehalten wird, und insbesondere eines Lagerelements, das in der Haltestellung mit mehreren Haltegliedern im Eingriff oder zwischen mehreren Haltegliedern eingeklemmt ist.

Bei einer bevorzugten Ausführungsform der Erfindung kann das mindestens eine Halteelement in Form eines Vorsprungs ausgebildet sein. Ein oder mehrere Vorsprünge sind an einem Halteglied auf einfache Weise ausbildbar.

Gemäß einer weiteren bevorzugten Ausführungsform der Erfindung kann das mindestens eine Halteelement in Form einer Ausnehmung ausgebildet sein. Auch eine Ausnehmung ist auf einfache Art und Weise an einem Halteglied vorsehbar.

Vorteilhafterweise ist mindestens ein Halteglied in Form einer profilierten Schiene ausgebildet. In diesem Fall kann ein Lagerelement in der Haltestellung an einer über die gesamte Längserstreckung der Schiene verlaufenden, strukturierten Oberfläche der Schiene stabil gehalten werden.

Günstig ist es, wenn das mindestens eine Lagerelement mindestens ein Verbindungselement aufweist, welches in der Haltestellung form- und/oder kraftschlüssig an dem mindestens einen Halteteil gehalten ist. Auf diese Weise kann das mindestens eine Lagerelement so ausgebildet werden, dass es in der Haltestellung besonders stabil an dem mindestens einen Halteteil gehalten ist.

Insbesondere ist es günstig, wenn das mindestens eine Verbindungselement zu einem Halteelement des Halteteils korrespondierend ausgebildet ist. Dadurch können in der Haltestellung jeweils ein Verbindungselement und ein Halteelement form- und/oder kraftschlüssig miteinander verbunden werden.

Vorzugsweise ist das mindestens eine Verbindungselement in Form eines Vorsprungs oder einer Ausnehmung ausgebildet. Ein in Form eines Vorsprungs ausgebildetes Verbindungselement eines Lagerelements kann beispielsweise zu einem in Form einer Ausnehmung ausgebildeten Halteelement eines Halteglieds korrespondierend ausgebildet sein.

Vorteilhafterweise ist das mindestens eine Verbindungselement in Form einer Nut ausgebildet. Eine Nut kann auf einfache Weise hergestellt und mit einem langgestreckten Vorsprung oder auch einer Mehrzahl von Vorsprüngen in Eingriff gebracht werden.

Insbesondere ist das mindestens eine Lagerelement vorteilhafterweise in Form einer Profilleiste ausgebildet. Eine Profilleiste weist eine Mehrzahl von Oberflächen auf, an denen verschiedenartig ausgeformte chirurgische Instrumente und/oder Implantate gehalten werden können.

Bei einer bevorzugten Ausführungsform der Erfindung können der mindestens eine Halteteil und die mindestens zwei Befestigungsglieder unlösbar verbunden sein. Dadurch wird die Anzahl der zum Zusammensetzen einer erfindungsgemäßen chirurgischen Halterung benötigten Einzelteile reduziert.

Bei einer bevorzugten Ausführungsform der Erfindung kann die Halterung mindestens teilweise aus einem Metall hergestellt sein. Beispielsweise können mindestens ein Halteteil und die Befestigungseinrichtung auf einfache Weise einstückig hergestellt werden, indem in einem Stanzschritt eine Vorlage aus einem flachen Blech gestanzt wird und die Vorlage in anschließenden Verformungsschritten in die gewünschte Form gebogen oder gefaltet wird. Wenn das Blech aus einem elastischen Metallwerkstoff besteht, kann auch ein in Form eines elastisch federnden Abschnitts eines Befestigungsgliedes ausgebildetes Rückstellelement der Halterung aus dem Blech hergestellt sein.

Bei einer weiteren vorteilhaften Ausführungsform der Erfindung kann die Halterung mindestens teilweise aus einem Kunststoff hergestellt sein. Wenn beispielsweise ein Lagerelement aus einem Kunststoff hergestellt ist, wird die Gefahr verringert, dass ein chirurgisches Instrument und/oder Implantat beim Anbringen an das Lagerelement beschädigt, beispielsweise zerkratzt, wird.

Die eingangs gestellte Aufgabe wird ferner bei einem chirurgischen Behälter der eingangs beschriebenen Art erfindungsgemäß dadurch gelöst, dass mindestens eine chirurgische Halterung in Form einer der vorstehend beschriebenen chirurgischen Halterungen ausgebildet ist. In diesem Fall kann die Halterung zum Beispiel als Ganzes innerhalb des Aufnahmeraums des Behälters an eine Seitenwand oder den Boden herangeführt und dort auf einfache Weise befestigt werden. Auch ein häufig wiederholtes Anbringen und Abnehmen einer oder mehrerer, gegebenenfalls unterschiedlich ausgeformter, Halterungen ist möglich. Ferner ermöglicht dies eine Vielzahl möglicher Kombinationen zwischen unterschiedlichen Lagerelementen und Halteteilen. Beispielsweise kann ein Halteteil in Abhängigkeit von den zu haltenden Instrumenten und/oder Implantaten gezielt mit einem Lagerelement versehen werden, das zum Halten dieser Instrumente und/oder Implantate geeignet ist. Während das mindestens eine Lagerelement an dem mindestens einen Halteteil in der Haltestellung stabil gehalten werden kann, bietet die Überführbarkeit des mindestens einen Halteteils in die Entnahmestellung die Möglichkeit, die Vorgänge des Verbindens und des Lösens des mindestens einen Lagerelements und des mindestens einen Halteteils häufig zu wiederholen, ohne dass starke Verschleißerscheinungen auftreten.

Vorzugsweise ist die mindestens eine Halterung eine der oben beschriebenen Halterungen. Diese weisen die bereits oben im Zusammenhang mit den verschiedenen Ausführungsformen erläuterten Vorteile auf.

Vorteilhaft ist es, wenn der Behälter mindestens eine Befestigungsfläche zum Befestigen der Befestigungseinrichtung der mindestens einen Halterung am Behälter aufweist. Eine Befestigungsfläche kann beispielsweise korrespondierend zu einer an der Halterung vorgesehenen Kupplungsoberfläche ausgebildet sein, wodurch die Stabilität der Befestigung der Halterung am Behälter erhöht wird.

Bei einer bevorzugten Ausführungsform der Erfindung kann vorgesehen sein, dass der Behälter eine Mehrzahl von Durchbrechungen aufweist, die durch mindestens einen Steg voneinander getrennt sind, und dass die mindestens eine Befestigungsfläche durch mindestens einen Teil einer Oberfläche eines Stegs definiert wird. Ein Durchbrechungen aufweisender chirurgischer Behälter ist einfach und im Vergleich zu einem durchbrechungsfreien Behälter unter Materialersparnis herstellbar. Die Durchbrechungen können auch das Ablaufen von flüssigem Sterilisationsmedium ermöglichen, das sich nach einem Sterilisationsvorgang im Behälter befindet.

Vorzugsweise weist der mindestens eine Steg eine Breite auf, die einem Abstand zwischen einer ersten Kupplungsebene und einer zweiten Kupplungsebene der mindestens einen Halterung entspricht. Beim Befestigen einer Halterung am Behälter kann dann ein an einem Befestigungsglied der Halterung vorgesehenes Kupplungselement beispielsweise mit einem Steg so in Eingriff gebracht werden, dass zwei voneinander weg weisende Befestigungsflächen des Stegs an jeweils einer Kupplungsoberfläche des Kupplungselements anliegen. Durch Druck oder Zug, der in einer senkrecht oder im Wesentlichen senkrecht zu den Kupplungsebenen verlaufenden Richtung ausgeübt wird, ist das Kupplungselement dann kaum bewegbar und insbesondere vom Steg nicht abnehmbar.

Günstig ist es, wenn die Durchbrechungen einen rechteckigen Querschnitt aufweisen. Dadurch erhalten auf einen weiteren Steg weisende Oberflächen der Stege eine ebene Form, so dass verschiedene Teile dieser Oberflächen als Befestigungsflächen geeignet sind, an denen eine an der Befestigungseinrichtung vorgesehene Anlegefläche einer Halterung stabil anlegbar ist.

Um eine hohe Anzahl und eine übersichtliche Anordnung der Positionen zu erreichen, an denen eine Halterung am Behälter befestigbar ist, ist es von Vorteil, wenn die Durchbrechungen regelmäßig angeordnet sind.

Bei einer bevorzugten Ausführungsform der Erfindung kann der chirurgische Behälter als Sterilbehälter ausgebildet sein. An einem Sterilbehälter befestigte chirurgische Halterungen dienen zum Tragen von Instrumenten und/oder Implantaten, die in dem Sterilbehälter einem Sterilisationsvorgang unterzogen und/oder steril gelagert werden sollen.

Bei einer weiteren bevorzugten Ausführungsform der Erfindung ist der chirurgische Behälter als Siebkorb ausgebildet. An einer an einem Siebkorb befestigten Halterung gehaltene chirurgische Instrumente und/oder Implantate können in dem Siebkorb beispielsweise Reinigungsvorgängen unterzogen werden.

Die nachfolgende Beschreibung einer bevorzugten Ausführungsform der Erfindung dient im Zusammenhang mit der Zeichnung der näheren Erläuterung. Es zeigen:
- Figur 1:: eine perspektivische Ansicht eines erstens Ausführungsbeispiels von zwei, jeweils an einem Boden eines erfindungsgemäßen chirurgischen Behälters befestigbaren, erfindungsgemäßen chirurgischen Halterungen;
- Figur 2:: eine perspektivische Ansicht einer der Halterungen aus Figur 1;
- Figur 3:: eine perspektivische Ansicht einer Befestigungseinrichtung und eines mit der Befestigungseinrichtung verbundenen Halteteils einer Lagereinrichtung einer der Halterungen aus Figur 1;
- Figur 4:: eine perspektivische Ansicht der Halterungen aus Figur 1 beim Befestigen derselben am Boden;
- Figur 5:: eine perspektivische Ansicht einer der Halterungen aus Figur 1 beim Nachsichern am Boden;
- Figur 6:: eine perspektivische Ansicht von unten jeweils eines am Boden befestigten ersten Befestigungsglieds der Halterungen aus Figur 1;
- Figur 7:: eine perspektivische Ansicht von unten eines am Boden befestigten zweiten Befestigungsglieds einer der Halterungen aus Figur 1;
- Figur 8:: eine perspektivische Ansicht der Halterungen aus Figur 1 beim Abnehmen vom Boden;
- Figur 9:: eine perspektivische Ansicht einer der Halterungen aus Figur 1 im Zustand der Trennung des Lagerelements vom Halteteil;
- Figur 10:: eine perspektivische Ansicht einer der Halterungen aus Figur 1 beim Verbinden des Lagerelements und des Halteteils; und
- Figur 11:: eine perspektivische Ansicht einer der Halterungen aus Figur 1 beim Befestigen des Lagerelements am Halteteil.
- Figur 12:: eine perspektivische Ansicht eines zweiten Ausführungsbeispiels einer an einem Boden eines erfindungsgemäßen chirurgischen Behälters befestigten erfindungsgemäßen chirurgischen Halterung;
- Figur 13:: eine Ansicht einer Stirnseite der in Figur 12 dargestellten Halterung im Zustand der Trennung der Halterung vom Boden;
- Figur 14:: eine Ansicht einer Stirnseite der in Figur 12 dargestellten Halterung beim Befestigen derselben am Boden;
- Figur 15:: eine perspektivische Ansicht der in Figur 12 dargestellten Halterung beim Befestigen derselben am Boden;
- Figur 16:: eine Seitenansicht der am Boden befestigten Halterung aus Figur 12;
- Figur 17:: eine perspektivische Ansicht eines dritten Ausführungsbeispiels einer an einem Boden eines erfindungsgemäßen chirurgischen Behälters befestigten erfindungsgemäßen chirurgischen Halterung.

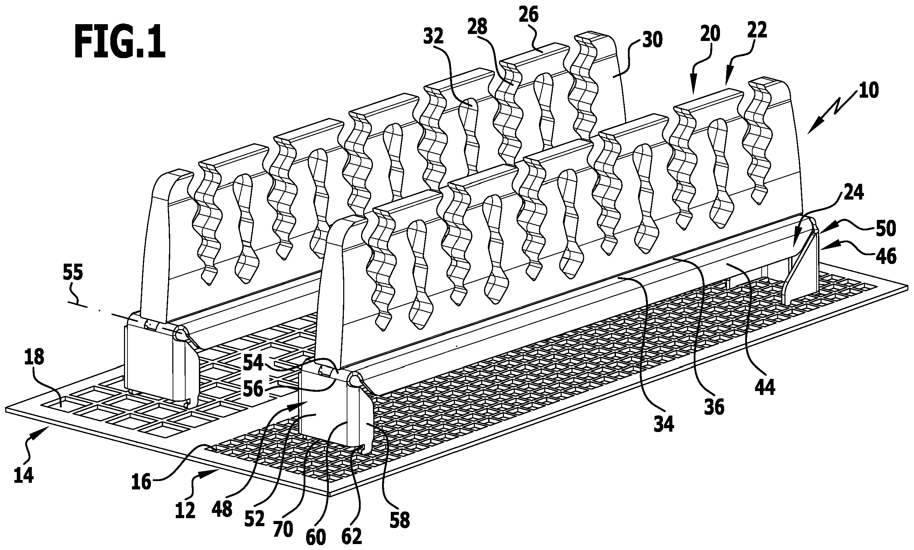

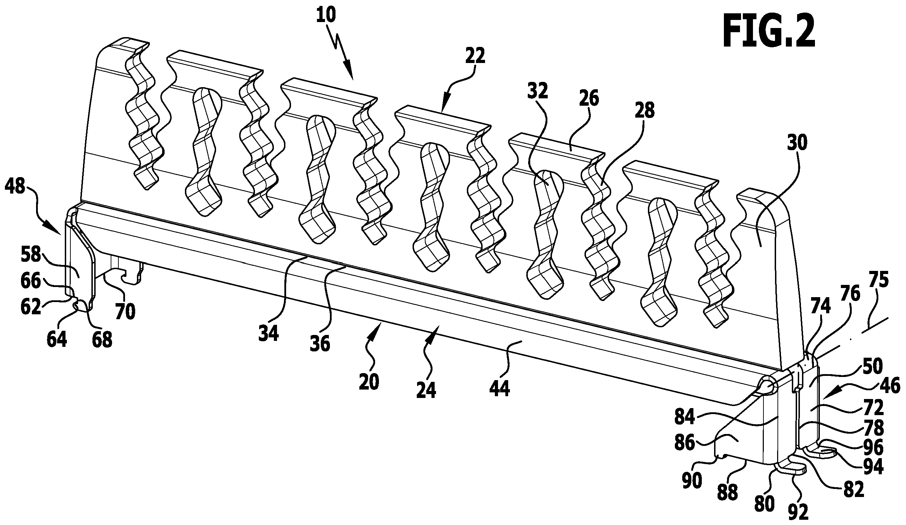

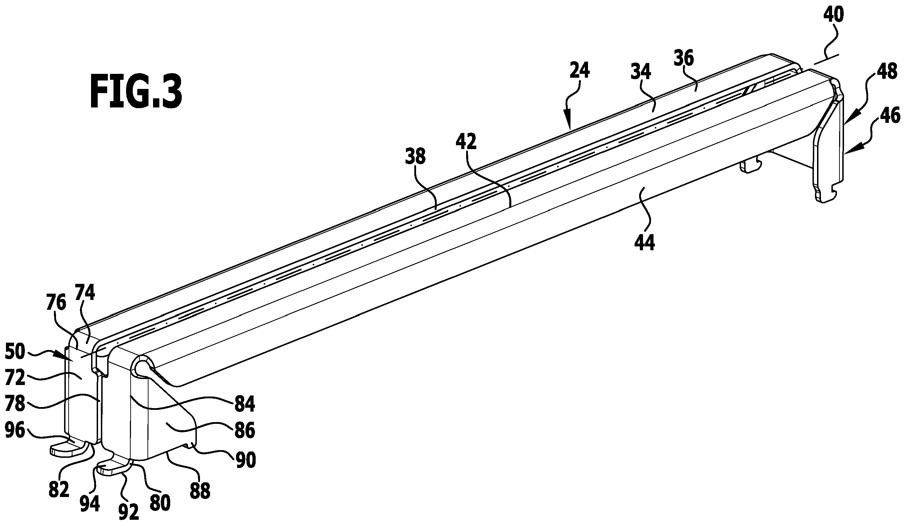

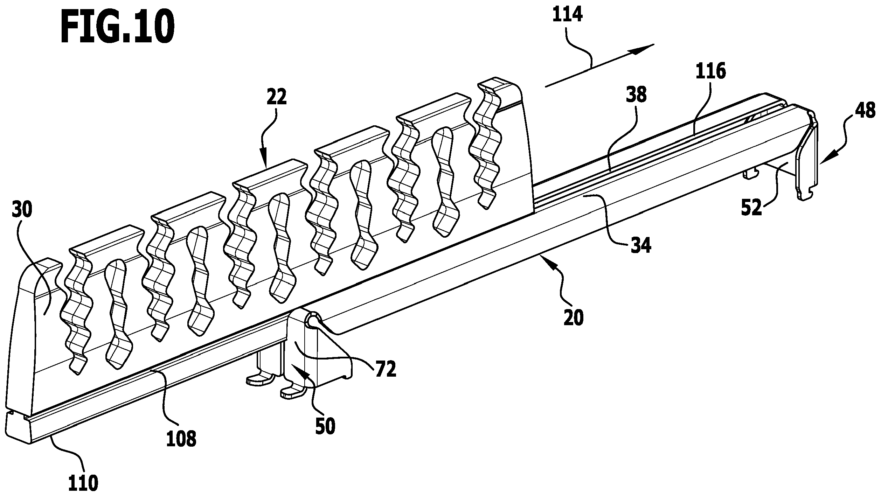

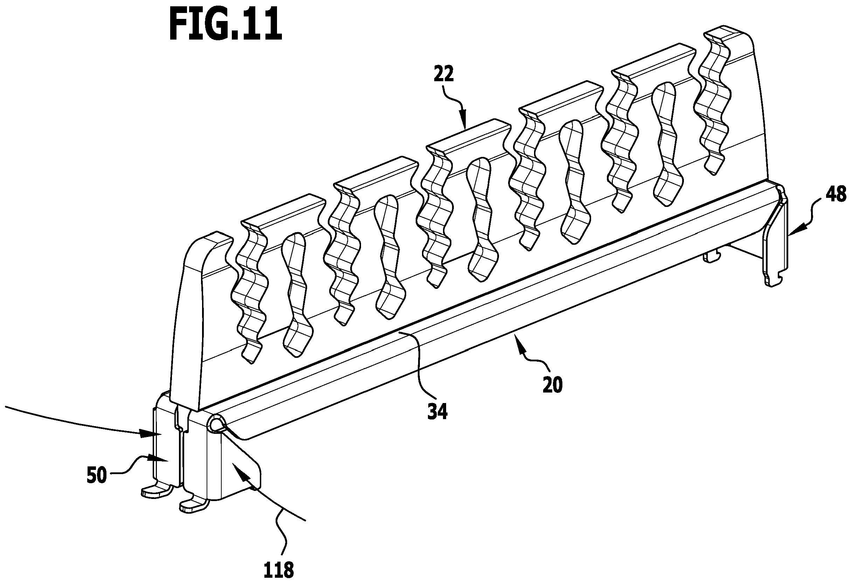

Im Zusammenhang mit den Figuren 1 bis 11 wird ein insgesamt mit dem Bezugszeichen 10 versehenes Ausführungsbeispiel einer erfindungsgemäßen chirurgischen Halterung zum Halten und/oder Lagern von chirurgischen Instrumenten und/oder Implantaten näher beschrieben. Die Halterung 10 kann an einem Boden oder einer Seitenwand eines erfindungsgemäßen chirurgischen Behälters befestigt werden. In den Figuren 1, 4, 6, 7 und 8 sind beispielhaft zwei Bodenabschnitte 12, 14 eines Bodens eines erfindungsgemäßen chirurgischen Behälters dargestellt, die mit regelmäßig angeordneten Durchbrechungen 16, 18 mit rechteckigem Querschnitt versehen sind, wobei der rechteckige Querschnitt der Durchbrechungen 18 des Bodenabschnitts 14 größer ist als der rechteckige Querschnitt der Durchbrechungen 16 des Bodenabschnitts 12. Die Halterung 10 kann an beiden Bodenabschnitten 16, 18 befestigt werden.

Eine Lagereinrichtung 20 der Halterung 10 umfasst ein Lagerelement 22 zum Lagern von chirurgischen Instrumenten und/oder Implantaten und einen Halteteil 24 zum Halten des Lagerelements 22. Das Lagerelement 22 ist aus einem Kunststoff hergestellt und als Profilleiste ausgebildet, die im Wesentlichen die Form eines flachen Quaders aufweist. Auf einer ersten Kantenfläche 26 ist das Lagerelement 22 mit einer Mehrzahl von Ausnehmungen 28 versehen, die sich jeweils zwischen voneinander weg weisenden Seitenflächen 30 des Lagerelements 22 erstrecken und parallel zu den Seitenflächen 30 einen profilierten Querschnitt aufweisen. In den Ausnehmungen 28 können chirurgische Instrumente und/oder Implantate gehalten und/oder gelagert werden.

Weiterhin ist das Lagerelement 22 mit Durchbrechungen 32 versehen, die sich wie die Ausnehmungen 28 jeweils zwischen den Seitenflächen 30 erstrecken. Die Ausnehmungen 28 sind in einer Längsrichtung des Lagerelements 22 bei einer gleichzeitigen Verengung der Durchbrechungen 32 elastisch spreizbar. Ein Instrument und/oder Implantat kann unter Spreizung einer Ausnehmung 28 in diese eingeführt und durch den durch die Elastizität des Lagerelements 22 bedingten Gegendruck stabil dort gehalten werden.

Der Halteteil 24 umfasst zwei Halteglieder 34 in Form ebener Abschnitte von zwei voneinander beabstandet parallel verlaufenden Schienen 36, die jeweils als langgestreckter, flacher quaderförmiger Streifen ausgebildet sind und zwischen denen in einer Haltestellung des Halteteils 24 das Lagerelement 22 gehalten werden kann. Auf das jeweils andere Halteglied 34 weisende Kantenflächen 38 jedes Haltegliedes 34 verlaufen parallel zueinander und zu einer zwischen ihnen verlaufenden Spiegelebene, wobei die Halterung 10 als Ganzes eine Spiegelsymmetrie bezüglich dieser Spiegelebene aufweist. Die Kantenflächen 38 definieren eine in der Spiegelebene liegende Längsachse 40 der Halterung 10. Von den Kantenflächen 38 weg weisende Seitenflächen 42 der Halteglieder 34 tragen jeweils über ihre gesamte Erstreckung eine Flügelplatte 44, die im Wesentlichen die Form eines Rechtecks aufweist, dessen freien Enden abgeflacht sind. Die Flügelplatten 44 sind von den Haltegliedern 34 in Richtung aufeinander zu abgebogen und liegen annähernd parallel zu der Spiegelebene.

Zum Befestigen der Halterung 10 an einem Behälter weist sie eine Befestigungseinrichtung 46 auf, die ein erstes Befestigungsglied 48 und zwei zweite Befestigungsglieder 50 umfasst. Das erste Befestigungsglied 48 umfasst eine annähernd quadratische ebene Grundplatte 52, die im Wesentlichen senkrecht zur Achse 40 verläuft, und jeweils einen ersten Endabschnitt 54 jeder Schiene 36. Die ersten Endabschnitte 54 der Schienen 36 sind um eine senkrecht zu der Spiegelebene verlaufende Querachse 55 in Richtung auf die Flügelplatten 44 der Halteglieder 34 zu von den Haltegliedern 34 abgebogen und jeweils an einer Stirnfläche voneinander versetzt mit einer Oberseite 56 der Grundplatte 52 verbunden. Somit sind die zwei Halteglieder 34 über das erste Befestigungsglied 48 miteinander verbunden. Das erste Befestigungsglied 48 ist somit von dem Halteteil 24 um einen Gliedwinkel von circa 90° abgewinkelt.

Weiterhin umfasst das erste Befestigungsglied 48 zwei annähernd rechteckige Flügelplatten 58, die jeweils auf einer von zwei zu der Oberseite 56 senkrecht verlaufenden Seiten 60 der Grundplatte 52 angebracht sind, von der Grundplatte 52 in Richtung auf die Schienen 36 abgebogen sind und annähernd parallel zu der Spiegelebene liegen. In einer von den Schienen 36 weg weisenden Richtung stehen die Flügelplatten 58 des ersten Befestigungsglieds 48 über seine Grundplatte 52 vor. In ihrem über die Grundplatte 52 vorstehenden Abschnitt ist an einer an die Grundplatte 52 angrenzenden Seite jeweils ein Kupplungselement 62 in Form einer Ausnehmung vorgesehen. Jede Ausnehmung weist eine erste Seitenfläche 64, die auf die Schienen 36 hin weist, eine zweite Seitenfläche 66, die von den Schienen 36 weg weist, und eine Grundfläche 68 auf. Die zweite Seitenfläche 66 liegt in einer Ebene mit einer ebenfalls von den Schienen 36 weg weisenden Unterseite 70 der Grundplatte 52. Die erste Seitenfläche 64 bildet eine erste Kupplungsoberfläche, die zweite Seitenfläche 66 eine zweite Kupplungsoberfläche und die Grundfläche 68 eine Anlegefläche des Kupplungselements 62.

Eine auf die Schienen 36 hin weisende Ecke der Flügelplatten 58 des ersten Befestigungsgliedes 48 ist abgeflacht, so dass sie und die ihr benachbarte, ebenfalls abgeflachte Ecke einer Flügelplatte 44 eines Haltegliedes 34 sich nicht gegenseitig behindern. Auch die von den Schienen 36 und der Grundplatte 52 weg weisenden Ecken der Flügelplatten 58 sind abgeflacht, um das Befestigen des ersten Befestigungsgliedes 48 an einem Behälter zu erleichtern.

Die zweiten Befestigungsglieder 50 umfassen jeweils eine senkrecht zu der Achse 40 verlaufende rechteckige Grundplatte 72 und den zweiten Endabschnitt 74 einer Schiene 36, wobei der zweite Endabschnitt 74 jeweils um eine senkrecht zu der Spiegelebene verlaufende Querachse 75 in Richtung auf die Flügelplatten 44 der Halteglieder 34 zu von dem Halteglied 34 abgebogen und an einer Stirnfläche mit einer oberen Schmalseite 76 der jeweiligen Grundplatte 72 verbunden ist. Somit sind die zweiten Befestigungsglieder 50 um einen Gliedwinkel von jeweils circa 90° von dem Halteteil 24 abgewinkelt. In der Haltestellung der Lagereinrichtung 20 berühren sich aufeinander zu weisende Längsseiten 78 der beiden Grundplatten 72 der zweiten Befestigungsglieder 50. Der Freiraum zwischen den beiden Schienen 36 wird durch Ausnehmungen in den Grundplatten 72 fortgesetzt.

Eine Rückfläche der Grundplatte 72 jedes zweiten Befestigungsglieds 50 trägt einen flachen Vorsprung 80, der abschnittsweise über eine der oberen Schmalseite 76 gegenüberliegende untere Schmalseite 82 des zweiten Befestigungsglieds 50 vorsteht und dessen freies Ende annähernd rechtwinklig in der vom ersten Befestigungsglied 48 weg weisenden Richtung abgewinkelt ist. Das freie Ende des flachen Vorsprungs 80 verläuft parallel zu den Schienen 36.

Die von dem jeweils anderen zweiten Befestigungsglied 50 weg weisende Längsseite 84 der Grundplatte 72 jedes zweiten Befestigungsgliedes 50 bildet eine erste Kathetenseite einer von der Grundplatte 72 in Richtung auf das erste Befestigungsglied zu abgewinkelten, annähernd parallel zu der Spiegelebene liegenden Flügelplatte 86, die die Form eines rechtwinkligen Dreiecks aufweist und deren Hypotenusenseite auf die benachbarte Schiene 36 zu weist. Eine zweite Kathetenseite 88 der Flügelplatte 86 verläuft in einer Ebene mit der unteren Schmalseite 82 der Grundplatte 72 und weist an ihrem freien Ende einen von den Schienen 36 weg weisenden Vorsprung 90 auf.

Der flache Vorsprung 80, der die untere Schmalseite 82 umfassende Abschnitt der Grundplatte 72 und der die zweite Kathetenseite 88 umfassende Abschnitt der Flügelplatte 86 bilden ein Kupplungselement 92 des zweiten Befestigungsgliedes 50. Dabei bildet eine auf die Schienen 36 weisende Oberfläche 94 des freien Endes des flachen Vorsprungs 80 eine erste Kupplungsoberfläche, die untere Schmalseite 82 der Grundplatte 72 und die zweite Kathetenseite 88 der Flügelplatte 86 bilden eine zweite Kupplungsoberfläche, und eine vom ersten Befestigungsglied 48 weg weisende Oberfläche 96 des über die Grundplatte 72 vorspringenden Abschnitts des flachen Vorsprungs 80 bildet eine Anlegefläche des Kupplungselements 92.

Die Befestigungsglieder 48, 50 und die Halteglieder 34 sind demnach fest miteinander verbunden. Sie können einstückig gearbeitet sein und beispielsweise durch Verformung aus einer flachen Vorlage hergestellt werden, die in einem Stanzschritt aus einem flachen Blech erzeugt werden kann.

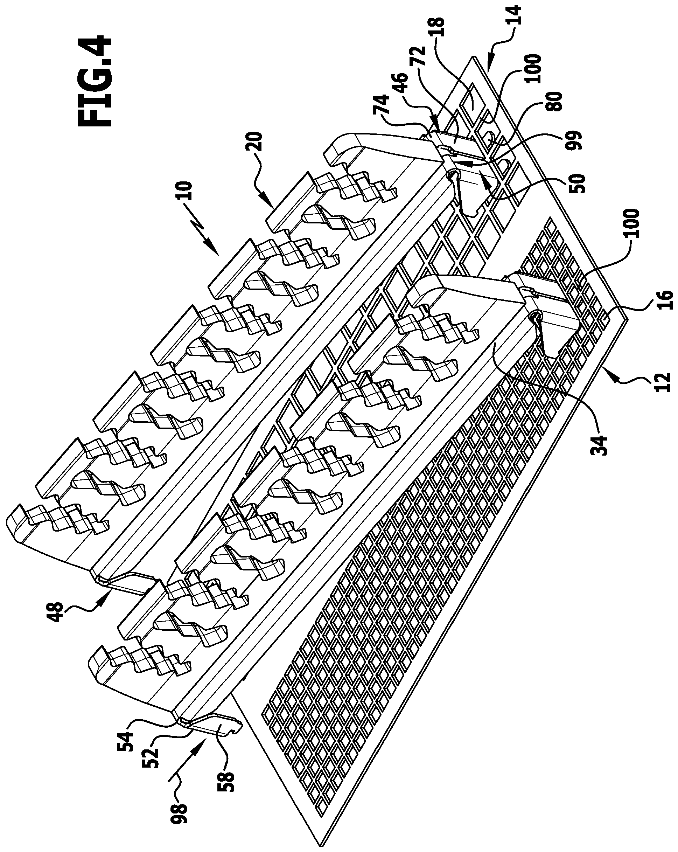

Um die Halterung 10 an einem Bodenabschnitt 12, 14 eines chirurgischen Behälters zu befestigen, können zunächst, wie in Figur 4 dargestellt, die zwei flachen Vorsprünge 80 der zweiten Befestigungsglieder 50 durch Durchbrechungen 16, 18 des jeweiligen Wandabschnitts 12, 14 geführt werden. Bei einem Befestigen an dem Wandabschnitt 14 mit größeren Durchbrechungen 18 werden die zwei flachen Vorsprünge 80 durch benachbarte Durchbrechungen 18 geführt, bei einem Befestigen an dem Wandabschnitt 12 mit kleineren Durchbrechungen 16 werden zwei Durchbrechungen 16 genutzt, zwischen denen sich eine weitere Durchbrechung 16 befindet.

Die Befestigungseinrichtung 46 kann von einer Befestigungsstellung in eine Anlegestellung überführt werden, indem auf die Grundplatte 52 des ersten Befestigungsgliedes 48 Druck in einer Richtung 98 parallel zu der Längsachse 40 auf die zweiten Befestigungsglieder 50 hin ausgeübt wird. Die Endabschnitte 54, 74 der Schienen 36 verbinden die Grundplatten 52, 72 der Befestigungsglieder 48, 50 jeweils elastisch federnd mit den Haltegliedern 34. Daher können die Befestigungsglieder 48, 50 durch Druck in der Richtung 98 relativ zu den Haltegliedern 34 ausgelenkt werden, wobei sich ein Abstand zwischen dem freien Ende des ersten Befestigungsgliedes 48 und den freien Enden der zweiten Befestigungsglieder 50 verringert. Durch die Verringerung des Abstands wird es ermöglicht, auch die über die Grundplatte 52 vorspringenden Abschnitte der Flügelplatten 58 des ersten Befestigungsgliedes 48 durch jeweils eine Durchbrechung 16, 18 zu führen. Insbesondere eine Führung durch eine der kleineren Durchbrechungen 16 des Wandabschnitts 12 wird dadurch erleichtert, dass die von den Schienen 36 und der Grundplatte 52 weg weisende Ecke der Flügelplatte 58 abgeflacht und der durch die Durchbrechung 16 zu führende Abschnitt der Flügelplatte 58 somit verschmälert ist.

Anschließend können die elastisch federnden Endabschnitte 54, 74 der Schienen 36 als Rückstellelemente genutzt werden. Sie definieren gemeinsam eine Rückstelleinrichtung 99 der Halterung 10. Sobald der Druck von der Grundplatte 52 des ersten Befestigungsgliedes 48 genommen wird, werden die Befestigungsglieder 48, 50 durch die Federkraft zurück in die Befestigungsstellung bewegt. Dabei gelangen alle vorhandenen Kupplungselemente in Eingriff mit jeweils zwischen zwei Durchbrechungen 16, 18 verlaufenden Stegen 100.

Die Elastizität der Rückstellelemente kann auf besonders einfache Weise sichergestellt werden, indem die Befestigungsglieder 48, 50 und die Halteglieder 34 durch Ausstanzen aus einem Federstahlblech und anschließendes Verformen einstückig ausgebildet werden.

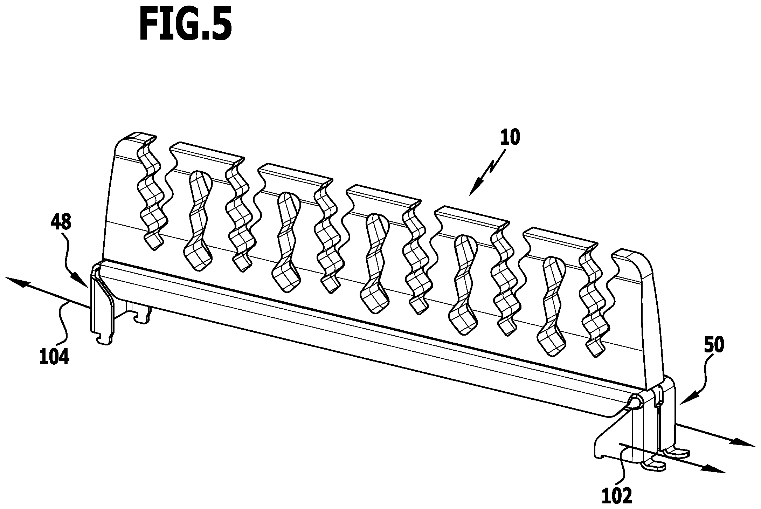

Die Überführung der Befestigungseinrichtung 46 aus der Anlegestellung in die Befestigungsstellung kann durch ein Nachsichern unterstützt werden, wie es in Figur 5 schematisch dargestellt ist. Auf das erste Befestigungsglied 48 wird ein von den zweiten Befestigungsgliedern 50 weg weisender Zug mit einer Richtung 102 parallel zu der Längsachse 40 ausgeübt, auf die zweiten Befestigungsglieder 50 ein vom ersten Befestigungsglied 48 weg weisender Zug mit einer Richtung 104 parallel zu der Längsachse 40.

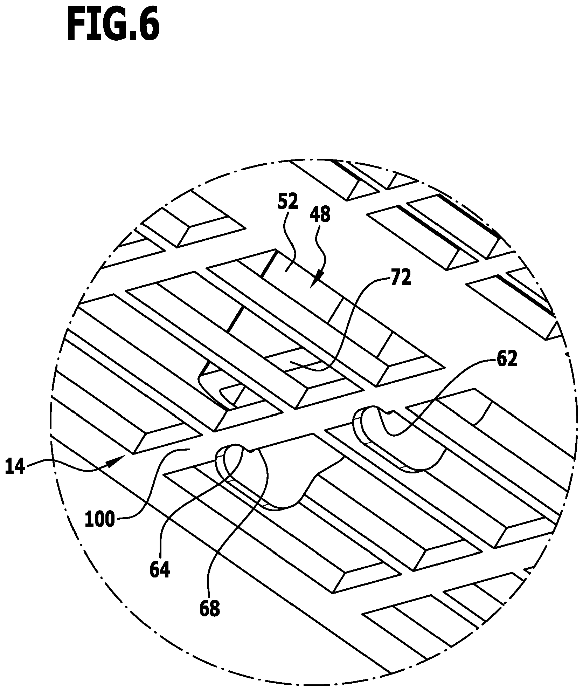

Wie in Figur 6 beispielhaft für eine an dem Bodenabschnitt 14 befestigte Halterung 10 dargestellt, ist jedes Kupplungselement 62 des ersten Befestigungsgliedes 48 in Eingriff mit einem Steg 100. Dabei liegen seine erste Seitenfläche 64, die die erste Kupplungsoberfläche definiert, an einer von den Schienen 36 weg weisenden Unterseite des Stegs 100 und seine zweite Seitenfläche 66, die die zweite Kupplungsoberfläche definiert, an einer auf die Schienen 34 hin weisenden Oberseite des Stegs 100 an. Zusätzliche Stabilität wird dadurch erhalten, dass auch die Unterseite 72 der Grundplatte 52 abschnittsweise auf einem weiteren Steg 100 aufliegt.

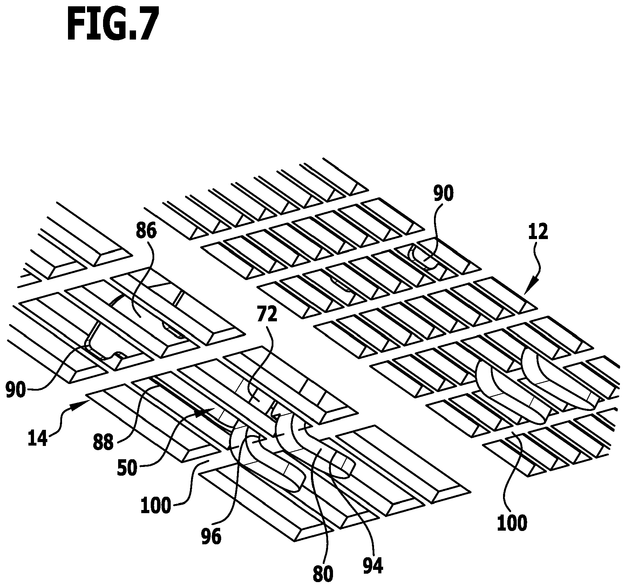

Wie in Figur 7 dargestellt, liegen bei einer am Behälter befestigten Halterung 10 weiterhin die Oberflächen 94 der freien Enden der flachen Vorsprünge 80 der zweiten Befestigungsglieder 50, die die ersten Kupplungsoberflächen definieren, jeweils an einer von den Schienen 34 weg weisenden Oberfläche eines Steges 100 an. An auf die Schienen 34 hin weisenden Oberflächen der Stege 100 liegen die unteren Schmalseiten 82 der Grundplatten 72 und die zweiten Kathetenseiten 88 der Flügelplatten 86 der zweiten Befestigungsglieder 50, die gemeinsam die zweiten Kupplungsoberflächen definieren, an. Durch diese Befestigung der Kupplungselemente der Halterung 10 an Stegen 100 ist ein Abnehmen der Halterung 10 vom jeweiligen Bodenabschnitt 12, 14 durch ein Ziehen an der Halterung 10 in einer zum jeweiligen Bodenabschnitt 12, 14 senkrecht verlaufenden Richtung nicht möglich.

Eine weiterhin erhöhte Stabilität der Befestigung der Halterung 10 am Behälter wird dadurch erreicht, dass die Befestigungsglieder 48, 50 jeweils eine Anlegefläche aufweisen, die in der Befestigungsstellung der Befestigungseinrichtung 46 an einer auf einen benachbarten Steg 100 weisenden Oberfläche eines Steges 100 anliegt. Im Fall des ersten Befestigungsgliedes 48 ist die Anlegefläche die Grundfläche 68 des Kupplungselements 62, im Falle der zweiten Befestigungsglieder 50 die vom ersten Befestigungsglied 48 weg weisende Oberfläche 96 des flachen Vorsprungs 80. Jede Anlegefläche sichert die Halterung 10 gegen ein Rutschen oder Gleiten in einer auf die jeweils anliegende Stegoberfläche hin weisenden Richtung. Da die Anlegeflächen des ersten Befestigungsgliedes 48 und die Anlegeflächen der zweiten Befestigungsglieder 50 in entgegengesetzte Richtungen weisen, ist eine Bewegung der Halterung 10 durch einen entlang der Achse 40 ausgeübten Zug oder Druck erschwert, solange die Befestigungseinrichtung in der Befestigungsstellung verbleibt.

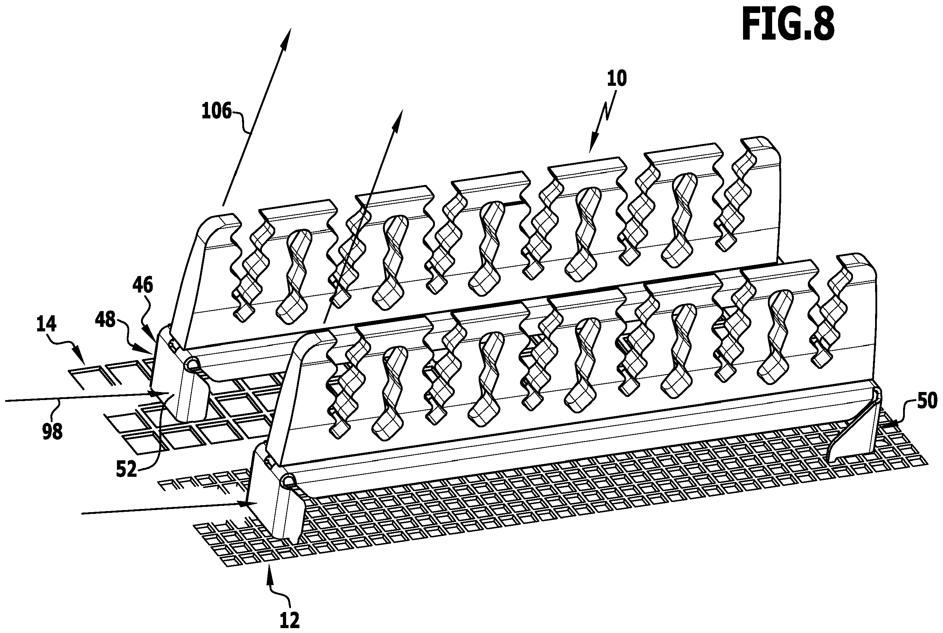

Um die Halterung 10 vom Behälter abnehmen zu können, muss zunächst wieder, wie in Figur 8 dargestellt, die Befestigungseinrichtung 46 aus der Befestigungsstellung in die Anlegestellung überführt werden, indem Druck in der Richtung 98 auf die Grundplatte 52 des ersten Befestigungsgliedes 48 ausgeübt wird. Während sich die Befestigungseinrichtung in der Anlegestellung befindet, können anschließend durch einen Zug an der Halterung 10 in einer vom jeweiligen Wandabschnitt 12, 14 weg weisenden Richtung 106 zunächst das erste Befestigungsglied 48 und nachfolgend die zweiten Befestigungsglieder 50 vom Behälter abgenommen werden.

Insbesondere bei einer Befestigung an einem Bodenabschnitt 12 mit kleineren Durchbrechungen 16 ist die Halterung 10 durch die an den Flügelplatten 86 der zweiten Befestigungsglieder 50 vorgesehenen Vorsprünge 90 davor geschützt, durch einen Druck auf die zweiten Befestigungsglieder 50 in Richtung auf das erste Befestigungsglied 48 hin in die Anlegestellung überführt und somit vom Behälter abnehmbar zu werden. Wie beispielsweise in Fig. 6 gezeigt, greifen die Vorsprünge 90 in jeweils eine Durchbrechung 16. Die zweiten Befestigungsglieder 50 können nicht so weit in Richtung des ersten Befestigungsgliedes 48 ausgelenkt werden, dass die flachen Vorsprünge 80 durch Durchbrechungen 16 herausgeführt werden könnten, da zuvor der Vorsprung 90 an einem Steg 100 anstößt. Somit muss zuerst das erste Befestigungsglied 48 von dem Bodenabschnitt 12 abgenommen werden.

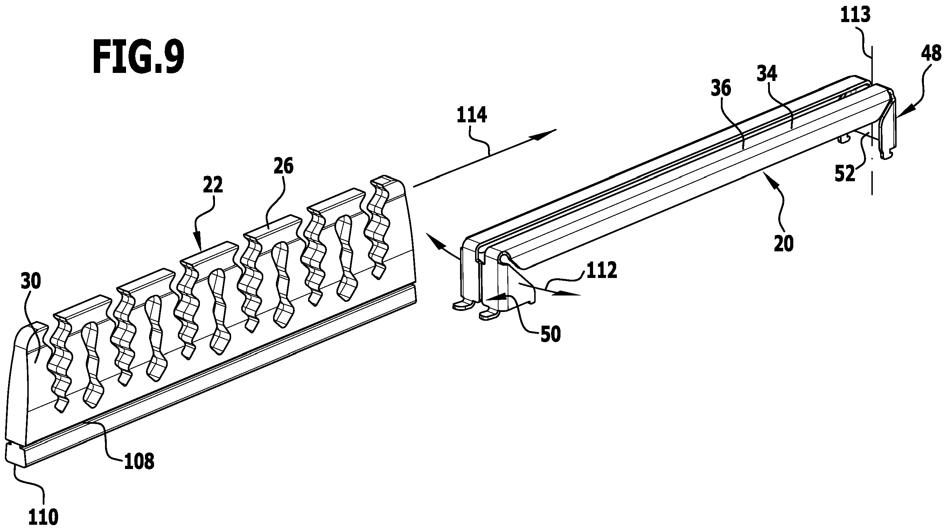

Die Figuren 9 bis 11 zeigen den Vorgang der Befestigung des Lagerelementes 22 an der zunächst in einer Haltestellung vorliegenden Lagereinrichtung 20.

Das Lagerelement 22 weist an seinen Seitenflächen 30 jeweils ein über eine gesamte Länge der Seitenfläche 30 verlaufendes Verbindungselement in Form einer Nut 108 auf. Beide Nuten 108 verlaufen in einem gleichen Abstand parallel zu einer von der ersten Kantenfläche 26 weg weisenden zweiten Kantenfläche 110 des Lagerelements 22.

Die Lagereinrichtung 20 wird aus der Haltestellung in eine Entnahmestellung gebracht, indem die beiden zweiten Befestigungsglieder 50 in Richtungen 112 voneinander weg bewegt und um eine durch die Grundplatte 52 des ersten Befestigungsglieds 48 verlaufende und in der Spiegelebene der Halterung 10 liegende Biegeachse 113 verschwenkt werden. Dadurch wird auch der Abstand zwischen den beiden Schienen 36 und somit zwischen den beiden Haltegliedern 34 vergrößert. Die Grundplatte 52 des ersten Befestigungsgliedes 48 wird dabei entlang der Biegeachse 113 leicht verbogen.

Das Lagerelement 22 kann nun mit einer Richtung 114 parallel zu der Achse 40 zwischen die zwei Halteglieder 34 geführt werden. Dabei wird jeweils eine Nut 108 vor einen mit der Kantenfläche 38 versehenen, als Halteelement ausgebildeten Kantenabschnitt 116 eines Haltegliedes 34 geführt. Die zwischen den Nuten 108 und der zweiten Kantenfläche 110 liegenden Abschnitte der Seitenflächen 30 des Lagerelements 22 werden dabei durch einen Freiraum zwischen den zwei Grundplatten 72 der zweiten Befestigungsglieder 50 geführt.

Während die Lagereinrichtung 20 noch die Entnahmestellung einnimmt, ist das Lagerelement 22 in lockeren Eingriff mit den Haltegliedern 34 bringbar. Somit kann es in der Richtung 114 auf einfache Weise verschoben, aber nur schwer in andere Richtungen ausgelenkt werden.

Wenn das Lagerelement 22 über die gesamte Erstreckung seiner Länge zwischen den Haltegliedern 34 positioniert ist, weisen die Kantenflächen 38 der Kantenabschnitte 116 jeweils auf eine Seitenfläche 30 des Lagerelements 22. Nun kann die Lagereinrichtung 20 durch eine Verschwenkbewegung der zweiten Befestigungsglieder 50 aufeinander zu, jeweils in einer Richtung 118, wieder in die Haltestellung überführt werden. Die Kantenabschnitte 116 greifen dann form- und kraftschlüssig in die Nuten 108 ein und das Lagerelement 22 ist in der Lagereinrichtung 20 befestigt.

Zur Abnahme des Lagerelements 22 aus dem Halteteil 24 muss dieser zunächst wieder in die Entnahmestellung überführt werden. Dies wird erschwert beziehungsweise ist möglich, wenn die Halterung 10 an einem Behälter befestigt ist. Da in diesem Fall die Befestigungsglieder 48, 50 mit Stegen 100 des Behälters in Eingriff sind, wird eine Bewegung der zweiten Befestigungsglieder 50 relativ zueinander ebenso erschwert beziehungsweise verhindert wie ein Verbiegen der Grundplatte 72 des zweiten Befestigungsgliedes 50. Somit ist das Lagerelement 22 im Halteteil 24 sicher gehalten, wenn die Halterung 10 an einem Behälter befestigt ist.

Anders als beispielsweise die in der DE 20 2005 015 415 U1 beschriebene Halterung, bei deren Anbringen an einem Behälter ein Befestigungselement von einer Außenseite des Behälters und ein Profilelement von einer Innenseite desselben an einen Boden des Behälters herangeführt und erst dann miteinander verbunden werden, kann die Halterung 10 von einer Seite eines Bodenabschnitts 12 oder 14, also beispielsweise innerhalb des Aufnahmeraums des Behälters, an den Bodenabschnitt 12 oder 14 herangeführt und an ihm als Ganzes, also mit einem mit dem Halteteil 24 verbundenen Lagerelement 22, angebracht werden, wie in Figur 4 gezeigt. Dabei ist das bevorzugt aus einem Kunststoff hergestellte Lagerelement an den mit den Befestigungsgliedern 48, 50 fest verbundenen, bevorzugt einstückig mit den Befestigungsgliedern 48, 50 aus einem rostfreien Federstahlblech hergestellten, Haltegliedern 34 befestigt. Üblicherweise werden beim Anbringen der Halterung 10 die zweiten Befestigungsglieder 50 mit einer Hand und das erste Befestigungsglied 48 mit einer anderen Hand jeweils an den Bodenabschnitt 12 oder 14 geführt und dort befestigt. Weitere Handgriffe sind beim Anbringen der Halterung 10 nicht notwendig, insbesondere keine Verbindung mehrerer Einzelteile der Halterung 10 miteinander. Diese Vorteile ergeben sich insbesondere daraus, dass die Befestigungsglieder 48, 50 über die Lagereinrichtung 20 miteinander verbunden sind.

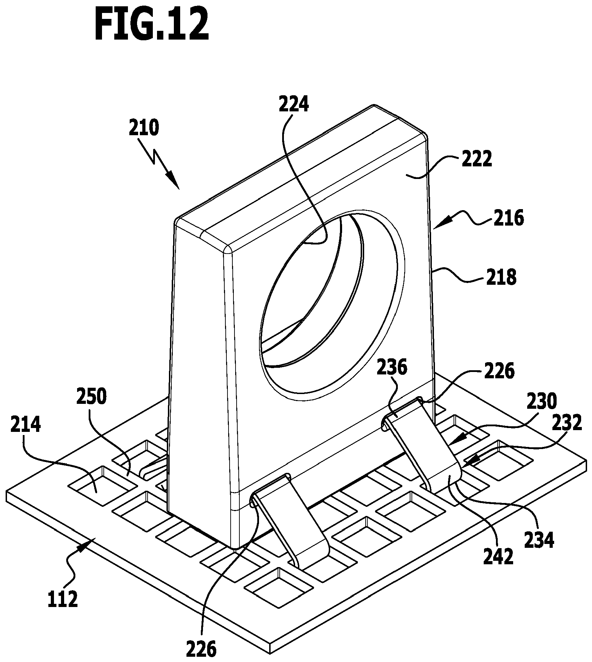

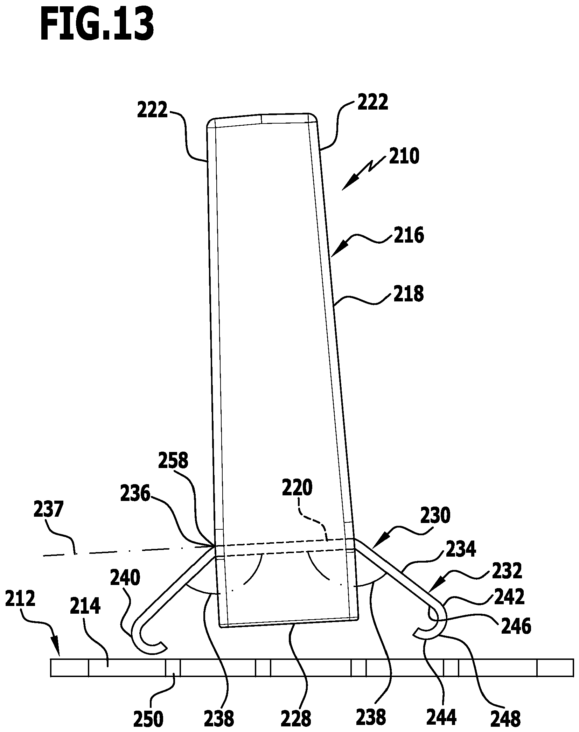

In den Figuren 12 bis 16 ist ein insgesamt mit den Bezugszeichen 210 versehenes zweites Ausführungsbeispiel einer erfindungsgemäßen chirurgischen Halterung zum Halten und/oder Lagern von chirurgischen Instrumenten und/oder Implantaten dargestellt. Weiterhin ist in den Figuren 12 bis 16 ein Bodenabschnitt 212 eines Bodens eines erfindungsgemäßen chirurgischen Behälters dargestellt, wobei die Halterung 210 an dem Bodenabschnitt 212 befestigt werden kann. Der Bodenabschnitt 212 ist mit regelmäßig angeordneten Durchbrechungen 214 mit rechteckigem Querschnitt versehen.

Eine Lagereinrichtung 216 der Halterung 210 umfasst ein Lagerelement 218 zum Halten und/oder Lagern von chirurgischen Instrumenten und/oder Implantaten und einen Halteteil 220 zum Halten des Lagerelements 218. Das Lagerelement 218 ist aus einem Kunststoff, beispielsweise Silikon, hergestellt und weist im Wesentlichen die Form eines flachen Quaders auf. Zwischen zwei voneinander weg weisenden Seitenflächen 222 des Lagerelements 218 erstreckt sich eine Durchbrechung 224 mit einem kreisförmigen Querschnitt. In der Durchbrechung 224 kann beispielsweise ein Implantat zu Lagerungszwecken aufgenommen werden.

Weiterhin weist das Lagerelement 218 zwei sich ebenfalls zwischen den Seitenflächen 222 erstreckende Halteteildurchbrechungen 226 mit einem Querschnitt in Form eines flachen Rechtecks auf, die von einer Kantenfläche 228 des Lagerelements 218 gleich weit beabstandet sind.

Jeder Halteteil 220 der Halterung 210 ist in Form eines Stegs ausgebildet und wird durch einen ebenen mittleren Abschnitt eines vorzugsweise aus einem Metall, insbesondere Federstahl, hergestellten Streifens 230 definiert. Jeweils ein Halteteil 220 ist durch eine Halteteildurchbrechung 226 des Lagerelements 218 geführt, so dass das Lagerelement 218 an den Halteteilen 220 gehalten ist.

An einem Behälter ist die Halterung 210 mittels einer Befestigungseinrichtung 232 befestigbar. Die Befestigungseinrichtung 232 umfasst vier Befestigungsglieder 234, wobei jeweils zwei Befestigungsglieder 234 über einen Halteteil 220 miteinander verbunden sind. Jedes Befestigungsglied 234 wird durch einen äußeren Abschnitt des Streifens 230 definiert und weist einen elastisch federnden Verbindungsabschnitt 236 auf, über den es mit dem Halteteil 220 verbunden ist. Vor den Seitenflächen 222 des Lagerelements 218 sind jeweils zwei Befestigungsglieder 234 positioniert.
Der ebene Halteteil 220 des Streifens 230 definiert eine Halteteilebene 237. Die beiden mit dem Halteteil 220 verbundenen Befestigungsglieder 234 sind von dem Halteteil 220 aus der Halteteilebene 237 heraus aufeinander zu abgebogen. Ein Gliedwinkel 238 zwischen dem Halteteil 220 und einem mit ihm verbundenen Befestigungsglied 234 beträgt jeweils circa 135°.

Jedes Befestigungsglied 234 umfasst ein freies Ende eines Streifens 230. Dieses freie Ende ist um ca. 180° in Richtung von der Halteteilebene 237 weg und auf das zweite mit demselben Halteteil 220 verbundene Befestigungsglied 234 hin umgebogen und definiert ein Kupplungselement 240, 242 des Befestigungsgliedes 234. Jedes Befestigungsglied 234 weist somit die Form eines Hakens auf, der zu dem zweiten mit demselben Halteteil 220 verbundenen Befestigungsglied 234 hin geöffnet ist.

Zwei aufeinander zu weisende Seitenflächen jedes Kupplungselements 240, 242 definieren zwei Kupplungsoberflächen 244, 246, wobei eine erste Kupplungsoberfläche 244 im Wesentlichen auf die Lagereinrichtung 216 hin weist und eine zweite Kupplungsoberfläche 246 im Wesentlichen von der Lagereinrichtung 216 weg weist.

Eine zwischen der ersten Kupplungsoberfläche 244 und der zweiten Kupplungsoberfläche 246 angeordnete, im Wesentlichen senkrecht zu der ersten Kupplungsoberfläche 244 und der zweiten Kupplungsoberfläche 246 verlaufende Oberfläche jedes Kupplungselements 240, 242 definiert eine Anlegefläche 248.

Der Halteteil 220 und die mit ihm verbundenen Befestigungsglieder 234 einschließlich der Kupplungselemente 240, 242 sind einstückig gearbeitet und können auf einfache Weise beispielsweise aus einem ebenen Metallstreifen durch mehrfaches Umbiegen erzeugt werden.

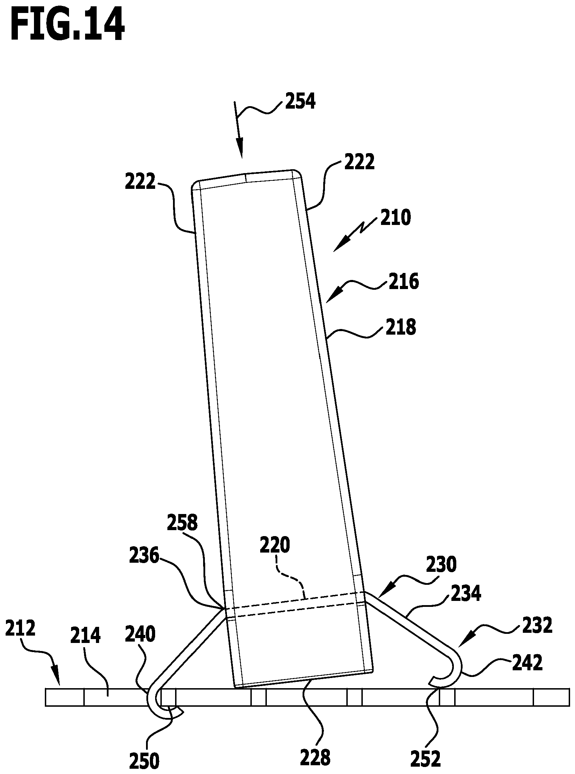

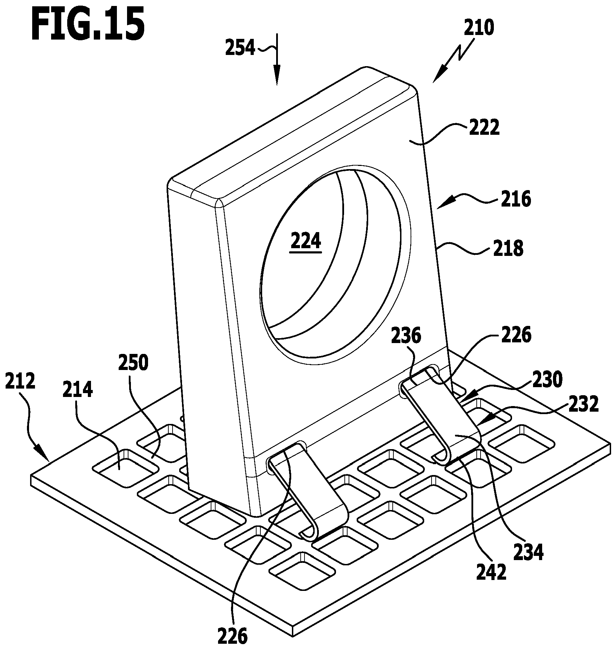

Zum Befestigen der Halterung 210 an dem Bodenabschnitt 212 eines chirurgischen Behälters können zunächst, wie in den Figuren 14 und 15 dargestellt, erste Kupplungselemente 240 der beiden vor einer der beiden Seitenflächen 222 des Lagerelements 218 angeordneten hakenförmigen Befestigungsglieder 234 durch jeweils eine Durchbrechung 214 des Bodenabschnitts 212 geführt werden. Die durch die Durchbrechung 214 geführten ersten Kupplungselemente 240 werden mit jeweils einem zwischen zwei Durchbrechungen 214 verlaufenden Steg 250 in Eingriff gebracht, so dass die erste Kupplungsoberfläche 240 an einer von der Lagereinrichtung 216 weg weisenden Unterseite des Stegs 250 und die zweite Kupplungsoberfläche 242 an einer auf die Lagereinrichtung 216 hin weisenden Oberseite des Stegs 250 anliegen. Die Anlegefläche 248 des Kupplungselements 240 liegt an einer auf einen benachbarten Steg 250 weisenden Oberfläche des Stegs 250 an. Die hakenförmigen Befestigungsglieder 234, die die ersten Kupplungselemente 240 umfassen, sind an jeweils einem Steg 250 eingehakt.

Zweite Kupplungselemente 242 der beiden vor der anderen Seitenfläche 222 des Lagerelements 218 angeordneten Befestigungsglieder 234 können nicht ebenso durch eine Durchbrechung 214 geführt werden, solange die Befestigungseinrichtung 232 in der Befestigungsstellung verbleibt, weil sie jeweils vor einem Steg 250 positioniert sind und nicht an diesem vorbeigeführt werden können.

Durch einen Druck auf die Befestigungsglieder 234 und/oder das Lagerelement 218 in einer Richtung 254 auf den Bodenabschnitt 212 hin kann die Befestigungseinrichtung 232 nun in die Anlegestellung überführt werden. Die bereits durch Durchbrechungen 214 geführten und an Stegen 250 anliegenden ersten Kupplungselemente 240 bleiben mit diesen Stegen 250 im Eingriff, während Außenflächen 252 der zweiten Kupplungselemente 243 über die Oberfläche des Stegs 250 gleiten, vor dem sie jeweils angeordnet sind. Dabei werden die Gliedwinkel 238 und ein Abstand zwischen den jeweils zwei über einen Halteteil 220 miteinander verbundenen Befestigungsgliedern 234 vergrößert. Durch diesen Prozess wird die Befestigungseinrichtung 232 in die Anlegestellung überführt, in der schließlich auch die zweiten Kupplungselemente 242 durch jeweils eine Durchbrechung 214 geführt werden können.

Die beiden elastisch federnden Verbindungsabschnitte 236 der Befestigungsglieder 234 definieren jeweils ein Rückstellelement der Halterung 210 und somit gemeinsam eine Rückstelleinrichtung 258 der Halterung 210. Nach der Durchführung der zweiten Kupplungselemente 242 durch die Durchbrechungen 214 werden die Gliedwinkel 238 durch die Federkraft wieder verringert, der Abstand zwischen den zweiten Kupplungselementen 242 und den ersten Kupplungselementen 240 wird kleiner. Auch die zweiten Kupplungselemente 242 werden durch Einschnappen mit jeweils dem Steg 250 in Eingriff gebracht, an dem ihre Außenfläche 252 während des Befestigens abgeglitten ist. Auch die hakenförmigen Befestigungsglieder 234, die die zweiten Kupplungselemente 242 umfassen, sind jetzt an jeweils einem Steg 250 eingehakt.

Auf die beschriebene Weise lassen sich die hakenförmigen Befestigungsglieder 234 der Halterung 210 einfach mithilfe der Rückstelleinrichtung 258 mit Stegen 250 des Behälters verrasten. Während der Haken jedes Befestigungsglieds 234 bei der Halterung 210 zu dem zweiten mit demselben Halteteil 220 verbundenen Befestigungsglied 234 hin geöffnet ist, können bei weiteren Ausführungsbeispielen auch hakenförmige Befestigungsglieder vorgesehen sein, die jeweils von einem zweiten mit demselben Halteteil verbundenen Befestigungsglied weg weisend geöffnet sind. Eine derartige Ausführungsform einer Halterung kann beispielsweise an einem Behälter befestigt werden, indem Gliedwinkel in einer Anlagestellung gegenüber einer Befestigungsstellung verkleinert werden.

Die Stabilität der Befestigung der Halterung 210 an dem Behälter wird weiterhin dadurch erhöht, dass auch die Kantenfläche 228 des Lagerelements 218 an den Oberflächen einer Mehrzahl von Stegen 250 anliegt.

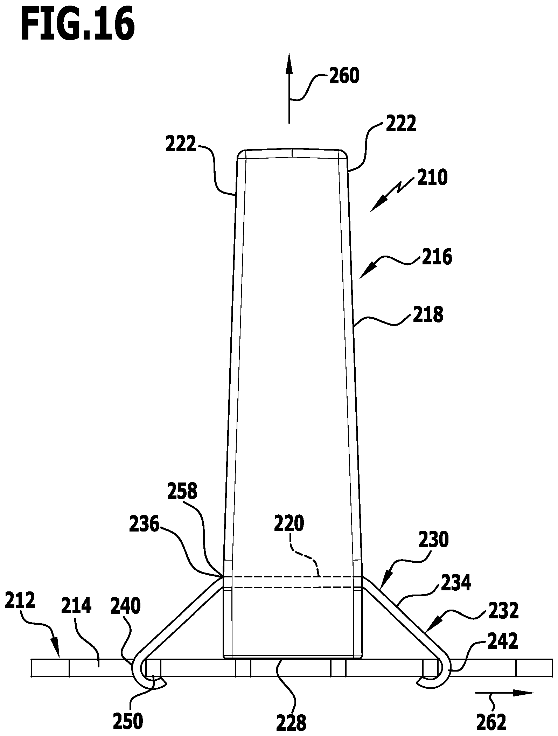

Wie in Figur 16 dargestellt, kann die an dem Bodenabschnitt 212 befestigte Halterung 210 durch einen Zug an dem Lagerelement 218 oder den Befestigungsgliedern 234 in einer vom Bodenabschnitt 212 weg weisenden Richtung 260 aufgrund des Anliegens der ersten Kupplungsoberflächen der Kupplungselemente 240, 242 an den Unterseiten der Stege 250 nicht vom Behälter abgenommen werden. Durch das Anliegen der Anlegeflächen 248 der Kupplungselemente 240, 242 an korrespondierenden Befestigungsflächen der Stege 250 ist die Halterung 210 auch gegen ein Rutschen oder Gleiten in einer auf die jeweilige Befestigungsfläche hin weisende Richtung geschützt.

Zum Abnehmen der Halterung 210 vom Bodenabschnitt 212 ist eine Überführung der Befestigungseinrichtung 232 aus der Befestigungsstellung in die Anlegestellung nötig. Dazu können die an Unterseiten von Stegen 250 anliegenden Teile beispielsweise der zweiten Kupplungselemente 242 in einer von den ersten Kupplungselementen 240 weg weisenden Richtung 262 verschoben werden, wobei die Position der ersten Kupplungselemente 240 unverändert bleibt und die Gliedwinkel 238 vergrößert werden. Nach Erreichen der Anlegestellung können die zweiten Kupplungselemente 242 durch die Durchbrechungen 214 geführt werden. Durch die Rückstelleinrichtung 258 wird die Befestigungseinrichtung 232 anschließend in die Befestigungsstellung zurück überführt, und unter Abnehmen auch der ersten Kupplungselemente 240 von dem jeweiligen Steg 250 und Durchführen der ersten Kupplungselemente 240 durch die Durchbrechungen 214 kann die Halterung 210 vom Bodenabschnitt 212 abgenommen werden.

Das Lagerelement 218 kann mit den Halteteilen 220 lösbar verbunden sein. Dann kann es von den Halteteilen 220 abgenommen werden, indem die Halteteile 220 aus den Halteteildurchbrechungen 226 herausgeführt werden. Dabei wird auch jeweils ein Befestigungsglied 234 mit dem an ihm vorgesehenen Kupplungselement 240, 242 durch die Halteteildurchbrechung 226 geführt.

Bei der Verbindung des Lagerelements 218 mit einem Halteteil 220 wird jeweils zunächst ein Befestigungsglied 234 durch eine Halteteildurchbrechung 226 geführt und anschließend der Halteteil 220 in die Halteteildurchbrechung 226 geführt.

Das Lagerelement 218 kann auch mit den Halteteilen 220 fest verbunden sein. In diesem Fall besteht die gesamte Halterung 210 aus einem einzigen Bauteil, das beispielsweise durch ein Umspritzen der Streifen 230 mit dem Kunststoff des Lagerelements 218 erhalten werden kann.

Der Streifen 230, dessen verschiedene Abschnitte bei der in Figur 12 bis Figur 16 dargestellten Halterung 210 den Halteteil 220 und die Befestigungseinrichtung 232 definieren, kann auch selbst eine vollständige erfindungsgemäße Halterung definieren. Im Unterschied zur Halterung 210 definiert sein mittlerer Abschnitt in diesem Fall keinen Halteteil 220 zum Halten eines Lagerelements, sondern dient selbst als Lagereinrichtung zum Halten und/oder Lagern von chirurgischen Instrumenten und/oder Implantaten. Diese einstückige Halterung kann ohne ein Zusammenwirken mit weiteren Bauteilen wie beispielsweise einem Lagerelement an einem Boden oder einer Seitenwand eines chirurgischen Behälters befestigt werden. Für das Befestigen und die Abnahme der Halterung am Behälter kann der gleiche Mechanismus genutzt werden wie bei der Halterung 210. Beispielsweise können zwischen der stegförmigen Lagereinrichtung dieser Halterung und einem Bodenabschnitt, an dem sie befestigt ist, chirurgische Instrumente, Implantate, Kabel, Platinen oder Kleinmotoren gehalten und/oder gelagert, beispielsweise eingeklemmt, werden. Somit sind die vorzugsweise aus Federstahl angefertigten Streifen 230 vielfältig einsetzbar.

Bei einer weiteren Ausführungsform der erfindungsgemäßen Halterung kann eine für das Halten und/oder Lagern chirurgischer Instrumente und/oder Implantate geeignete Lagereinrichtung durch eine weitere Verarbeitung des mittleren Abschnitts des Streifens 230, beispielsweise durch Verformen oder Umspritzen mit einem Kunststoff, erhalten werden.

Die Halterung 210 kann als Ganzes, also mit einem mit den Halteteilen 220 verbundenen Lagerelement 218, mit einem einfachen Handgriff an einem Bodenabschnitt 212 eines Behälters befestigt werden. Die Befestigungsglieder 234, die über die Lagereinrichtung 216 miteinander verbunden sind, können dabei von einer Seite des Bodenabschnitts 212 aus an diesen heran geführt und an ihm befestigt werden. Dabei werden zunächst die ersten Kupplungselemente 240 und anschließend die zweiten Kupplungselemente 242 mit jeweils einem Steg 250 in Eingriff gebracht. Diese Schritte können rasch aufeinander folgend mit einer Hand vorgenommen werden.

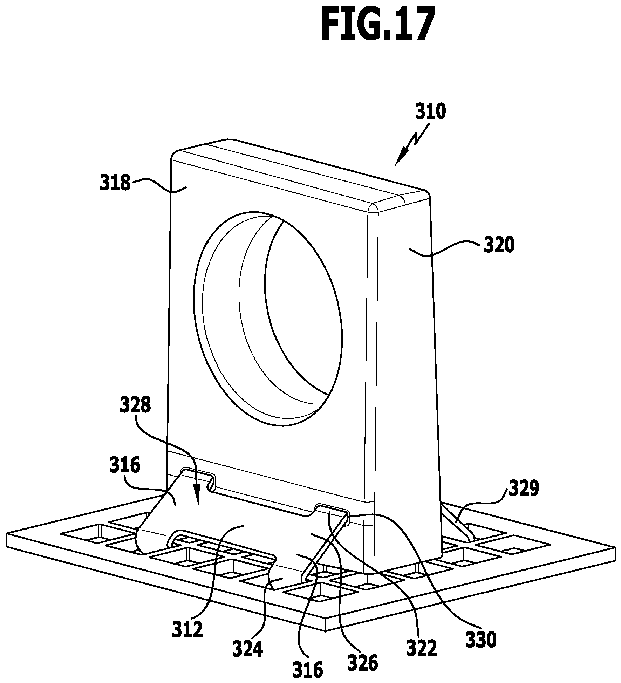

In Figur 17 ist ein drittes Ausführungsbeispiel einer erfindungsgemäßen chirurgischen Halterung für chirurgische Instrumente und/oder Implantate für einen chirurgischen Behälter dargestellt. Die verschiedenen Elemente, die die Halterung 310 umfasst, sind mit den Elementen der in Figur 12 bis in Figur 16 beschriebenen Halterung 210 identisch. Allerdings ist bei der Halterung 310 zusätzlich ein Stabilisierungselement 312 vorgesehen. Es verbindet zwei mit unterschiedlichen Halteteilen verbundene Befestigungsglieder 316 miteinander, wobei die verbundenen Befestigungsglieder 316 vor derselben Seitenfläche 318 eines an den Halteteilen gehaltenen Lagerelements 320 angeordnet sind. Das Stabilisierungselement 312 erstreckt sich zwischen aufeinander zu weisenden Seiten von jeweils zwischen einem Verbindungsabschnitt 322 und einem Kupplungselement 324 angeordneten Abschnitten 326 der zwei Befestigungsglieder 316. Anstelle der zwei voneinander getrennten Streifen 230 der Halterung 210 umfasst die Halterung 310 somit ein einstückig gearbeitetes Bauteil 328. Es umfasst zwei Halteteile, jeweils zwei mit jedem Halteteil verbundene Befestigungsglieder 316, 329 und das Stabilisierungselement 312. Das Bauteil 328 kann durch das Ausstanzen einer flachen Vorlage aus einem Blech und ein anschließendes Umbiegen der Vorlage hergestellt werden. Vorzugsweise ist es aus rostfreiem Federstahl hergestellt.

Das Lagerelement 320 kann mit dem Bauteil 328 lösbar verbunden sein. Das Lagerelement 320 kann dann an den Halteteilen gelagert werden, indem zwei nicht über das Stabilisierungselement 312 miteinander verbundene Befestigungsglieder 329 und anschließend die mit ihnen verbundenen Halteteile durch jeweils eine Halteteildurchbrechung 330 des Lagerelements 320 geführt werden. Ein Abnehmen des Lagerelements 320 von den Halteteilen 314 wird durch ein Herausführen zunächst der Halteteile 314 und anschließend der nicht über das Stabilisierungselement 312 miteinander verbundenen Befestigungsglieder 329 aus den Halteteildurchbrechungen 330 vorgenommen.

Die Verwendung des Bauteils 328 bietet den Vorteil, dass beim Befestigen des Lagerelements 320 an den Halteteilen mit nur zwei Einzelteilen gearbeitet werden kann und insbesondere beide Halteteile in einem Handgriff mit dem Lagerelement 320 verbunden werden können. Zudem ergibt sich eine höhere Stabilität der Befestigung der Halterung 310 an einem Behälter.

Alternativ dazu kann auch im Fall der Halterung 310 das Lagerelement 320 mit dem Bauteil 328 fest verbunden sein.

Das Bauteil 328 kann nicht nur in Verbindung mit einem Lagerelement 320 zusammen eine Halterung 310 bilden, sondern wie der oben beschriebene Streifen 230 auch eigenständig eine erfindungsgemäße Halterung definieren. Dabei werden die bei der Halterung 310 die Halteteile definierenden Abschnitte des Bauteils 328 selbst als Lagereinrichtung genutzt.

## Patentansprüche

1. Chirurgische Halterung (10; 210; 310) für chirurgische Instrumente und/oder Implantate für einen chirurgischen Behälter, insbesondere einen Sterilbehälter oder Siebkorb, umfassend eine Lagereinrichtung (20; 216) zum Halten und/oder Lagern von chirurgischen Instrumenten und/ oder Implantaten und eine Befestigungseinrichtung (46; 232) zum Befestigen der Halterung (10; 210; 310) am Behälter, wobei die Lagereinrichtung (20) mindestens ein Lagerelement (22) zum Halten und/oder Lagern von chirurgischen Instrumenten und/oder Implantaten und mindestens einen Halteteil (24) zum Halten des mindestens einen Lagerelements (22) umfasst und dass der mindestens eine Halteteil (24) von einer Haltestellung, in welcher das mindestens eine Lagerelement (22) an dem mindestens einen Halteteil (24) gehalten ist, in eine Entnahmestellung überführbar ist, in welcher das mindestens eine Lagerelement (22) und der mindestens eine Halteteil (24) voneinander lösbar sind, **gekennzeichnet durch** einen Halteteil (24) und dass der Halteteil (24) und die Befestigungseinrichtung einstückig gearbeitet sind.

2. Halterung nach Anspruch 1, **dadurch gekennzeichnet, dass** die Befestigungseinrichtung (46; 232) von einer Befestigungsstellung, in welcher sie mit dem Behälter in Eingriff ist und verbunden ist, in eine Anlegestellung überführbar ist, in welcher sie mit dem Behälter außer Eingriff ist, und dass die Befestigungseinrichtung (46; 232) mindestens zwei über die Lagereinrichtung (20; 216) miteinander verbundene Befestigungsglieder (48; 50; 234; 316; 329) zum Befestigen der Halterung (10; 210; 310) am Behälter umfasst.

3. Halterung nach Anspruch 2, **gekennzeichnet durch** eine Rückstelleinrichtung (99; 258) zum Überführen der Befestigungseinrichtung (46; 232) von der Anlegestellung in die Befestigungsstellung vorgesehen ist.

4. Halterung nach Anspruch 3, **dadurch gekennzeichnet, dass** die Rückstelleinrichtung (99; 258) mindestens ein Rückstellelement (54; 74; 236) zum Überführen der Befestigungseinrichtung (46; 232) von der Anlegestellung in die Befestigungsstellung umfasst, welches einem der mindestens zwei Befestigungsglieder (48; 50; 234; 316; 329) zugeordnet ist.

5. Halterung nach einem der Ansprüche 2 bis 4, **dadurch gekennzeichnet, dass** ein Abstand zwischen den mindestens zwei Befestigungsgliedern (48; 50; 234; 316; 329) in der Anlegestellung größer oder kleiner ist als in der Befestigungsstellung.

6. Halterung nach einem der Ansprüche 2 bis 5, **dadurch gekennzeichnet, dass** mindestens eines der mindestens zwei Befestigungsglieder (48; 50; 234; 316; 329) mindestens ein Kupplungselement (62; 92; 240; 242) umfasst, dass das mindestens eine Kupplungselement (62; 92; 240; 242) eine auf die Lagereinrichtung (20; 216) hin oder im Wesentlichen hin weisende erste Kupplungsoberfläche (64; 244) zum Anlegen an eine korrespondierende Befestigungsfläche eines chirurgischen Behälters aufweist, und dass die Halterung mindestens eine von der Lagereinrichtung (20; 216) weg oder im Wesentlichen weg weisende zweite Kupplungsoberfläche (66; 246) zum Anlegen an mindestens eine korrespondierende Befestigungsfläche eines chirurgischen Behälters aufweist.

7. Halterung nach einem der voranstehenden Ansprüche, **dadurch gekennzeichnet, dass** die Lagereinrichtung (20; 216) mindestens ein Lagerelement (22; 218) zum Halten und/oder Lagern von chirurgischen Instrumenten und/oder Implantaten und mindestens einen Halteteil (24; 220) zum Halten des mindestens einen Lagerelements (22; 218) umfasst und dass die Befestigungseinrichtung (46; 232) mindestens zwei über den mindestens einen Halteteil (24; 220) miteinander verbundene Befestigungsglieder (48; 50; 234; 316; 329) umfasst.

8. Halterung nach einem der voranstehenden Ansprüche, **dadurch gekennzeichnet, dass** der mindestens eine Halteteil (24) mindestens ein Halteglied (34) zum Halten des mindestens einen Lagerelements (22) in der Haltestellung umfasst.

9. Halterung nach Anspruch 8, **dadurch gekennzeichnet, dass** der mindestens eine Halteteil (24) mindestens zwei Halteglieder (34) umfasst und dass die mindestens zwei Halteglieder (34) relativ zueinander beweglich angeordnet sind.

10. Halterung nach Anspruch 9, **dadurch gekennzeichnet, dass** die mindestens zwei Halteglieder (34) relativ zueinander verschwenkbar angeordnet sind.

11. Halterung nach Anspruch 9 oder 10, **dadurch gekennzeichnet, dass** die mindestens zwei Halteglieder (34) jeweils ein erstes Ende und ein zweites Ende aufweisen und an ihren ersten Enden über eine Gelenkverbindung miteinander verbunden sind.

12. Halterung nach Anspruch 11, **dadurch gekennzeichnet, dass** die Gelenkverbindung in Form eines einfachen Scharniers, eines Filmscharniers oder eines Federscharniers ausgebildet ist und/oder dass ein Befestigungsglied (48) die Gelenkverbindung umfasst und/oder an dem zweiten Ende mindestens eines Haltegliedes (34) ein Befestigungsglied (50) angeordnet ist.

13. Halterung nach einem der Ansprüche 8 bis 12, **dadurch gekennzeichnet, dass** das mindestens eine Halteglied (34) mindestens ein Halteelement (116) zum form- und/oder kraftschlüssigen Halten des mindestens einen Lagerelements (22) in der Haltestellung aufweist.

14. Halterung nach einem der voranstehenden Ansprüche, **dadurch gekennzeichnet, dass** das mindestens eine Lagerelement (22) mindestens ein Verbindungselement (108) aufweist, welches in der Haltestellung form- und/oder kraftschlüssig an dem mindestens einen Halteteil (24) gehalten ist.

15. Chirurgischer Behälter zum Lagern und/oder Sterilisieren und/oder Reinigen von chirurgischen Instrumenten und/oder Implantaten, umfassend einen durch einen Boden und Seitenwände definierten Aufnahmeraum und mindestens eine chirurgische Halterung (10; 210; 310) für chirurgische Instrumente und/oder Implantate, **dadurch gekennzeichnet, dass** die mindestens eine Halterung (10; 210; 310) eine Halterung (10; 210; 310) gemäß einem der voranstehenden Ansprüche ist.

## Claims

1. Surgical holder (10, 210; 310) for surgical instruments and/or implants for a surgical container, in particular, a sterile container or perforated basket, comprising a storage device (20; 216) for holding and/or storing surgical instruments and/or implants and an attachment device (46; 232) for attaching the holder (10; 210; 310) to the container, wherein the storage device (20) comprises at least one storage element (22) for holding and/or storing surgical instruments and/or implants and at least one holding part (24) for holding the at least one storage element (22) and wherein the at least one holding part (24) is transferable from a holding position, the at least one storage element (22) being held on the at least one holding part (24) in said position, into a removal position, the at least one storage element (22) and the at least one holding part (24) being detachable from one another in said position, **characterized by** a holding part (24) and in that the holding part (24) and the attachment device are worked in one piece.

2. Holder according to claim 1, **characterized in that** the attachment device (46; 232) is transferable from an attaching position, said attachment device is engaged with and connected to the container in said position, into a position of abutment, said attachment device is disengaged with the container in said position, and **in that** the attachment device (46; 232) comprises at least two attachment members (48; 50; 234; 316; 329) for attaching the holder (10; 210; 310) to the container, said attachment members being connected to one another via the storage device (20; 216).

3. Holder according to claim 2, **characterized by** a restoring device (99; 258) for transferring the attachment device (46; 232) from the position of abutment into the attaching position.

4. Holder according to any one of claim 3, **characterized in that** the restoring device (99; 258) comprises at least one restoring element (54; 74; 236) for transferring the attachment device (46; 232) from the position of abutment into the attaching position, said element being associated with one of the at least two attachment members (48; 50; 234; 316; 329).

5. Holder according to any one of claims 2 to 4, **characterized in that** a distance between the at least two attachment members (48; 50; 234; 316; 329) is greater or smaller in the position of abutment than in the attaching position.

6. Holder according to claims 2 to 5, **characterized in that** at least one of the at least two attachment members (48; 50; 234; 316; 329) comprises at least one coupling element (62; 92; 240; 242), wherein the at least one coupling element (62; 92; 240; 242) has a first coupling surface (64; 244) for abutment on a corresponding attachment surface of a surgical container, said first coupling surface pointing towards or essentially towards the storage device (20; 216), and wherein the holder has at least one second coupling surface (66; 246) for abutment on at least one corresponding attachment surface of a surgical container, said second coupling surface pointing away or essentially away from the storage device (20; 216).

7. Holder according to any one of the preceding claims, **characterized in that** the storage device (20; 216) comprises at least one storage element (22; 218) for holding and/or storing surgical instruments and/or implants and at least one holding part (24, 220) for holding the at least one storage element (22; 218) and **in that** the attachment device (46; 232) comprises at least two attachment members (48; 50; 234; 316; 329) connected to one another via the at least one holding part (24; 220).

8. Holder according to any one of the preceding claims, **characterized in that** the at least one holding part (24) comprises at least one holding member (34) for holding the at least one storage element (22) in the holding position.

9. Holder according to claim 8, **characterized in that** the at least one holding part (24) comprises at least two holding members (34) and **in that** the at least two holding members (34) are arranged so as to be movable relative to one another.

10. Holder according to claim 9, **characterized in that** the at least two holding members (34) are arranged so as to be pivotable relative to one another.

11. Holder according to claims 9 or 10, **characterized in that** the at least two holding members (34) each have a first end and a second end and are connected to one another at their first ends via a hinged connection.

12. Holder according to claim 11, **characterized in that** the hinged connection is designed in the form of a simple hinge, a film hinge or a spring hinge and/or **in that** one attachment member (48) comprises the hinged connection and/or **in that** an attachment member (50) is arranged at the second end of at least one holding member (34).

13. Holder according to any one of claims 8 to 12, **characterized in that** the at least one holding member (34) has at least one holding element (116) for holding the at least one storage element (22) in the holding position in a form-locking and/or force-locking manner.

14. Holder according to any one of the preceding claims, **characterized in that** the at least one storage element (22) has at least one connecting element (108) held on the at least one holding part (24) in the holding position in a form-locking and/or force-locking manner.

15. Surgical container for the storage and/or sterilization and/or cleaning of surgical instruments and/or implants, comprising a receiving space defined by a base and side walls and at least one surgical holder (10; 210; 310) for surgical instruments and/or implants, **characterized in that** the at least one holder (10; 210; 310) is a holder according to any one of the preceding claims.

## Revendications

1. Système de maintien chirurgical (10 ; 210 ; 310) pour instruments chirurgicaux et/ou implants pour un contenant chirurgical, notamment un contenant stérile ou un panier à tamis, comprenant un dispositif de support (20 ; 216) pour maintenir et/ou supporter des instruments chirurgicaux et/ou des implants, et un dispositif de fixation (46 ; 232) pour fixer le système de maintien (10 ; 210 ; 310) au contenant, dans lequel le dispositif de support (20) comprend au moins un élément de support (22) pour maintenir et/ou supporter des instruments chirurgicaux et/ou des implants, et au moins une pièce de maintien (24) pour maintenir ledit au moins un élément de support (22), et en ce que ladite au moins une pièce de maintien (24) peut être transférée d'une position de maintien dans laquelle ledit au moins un élément de support (22) est maintenu sur ladite au moins une pièce de maintien (24), à une position de prélèvement dans laquelle ledit au moins un élément de support (22) et ladite au moins une pièce de maintien (24) peuvent être mutuellement désolidarisés, **caractérisé par** une pièce de maintien (24) et en ce que la pièce de maintien (24) et le dispositif de fixation sont travaillés d'une seule pièce.

2. Système de maintien selon la revendication 1, **caractérisé en ce que** le dispositif de fixation (46 ; 232) peut être transféré d'une position de fixation dans laquelle il est en prise et relié au contenant, à une position d'application dans laquelle il est hors de prise avec le contenant, et **en ce que** le dispositif de fixation (46 ; 232) comprend au moins deux organes de fixation (48 ; 50 ; 234 ; 316 ; 329) reliés mutuellement par le dispositif de support (20 ; 216) pour fixer le système de maintien (10 ; 210 ; 310) au contenant.

3. Système de maintien selon la revendication 2, **caractérisé par** un dispositif de rappel (99 ; 258) qui est prévu pour transférer le dispositif de fixation (46 ; 232) de la position d'application à la position de fixation.

4. Système de maintien selon la revendication 3, **caractérisé en ce que** le dispositif de rappel (99 ; 258) comprend au moins un élément de rappel (54 ; 74 ; 236) pour transférer le dispositif de fixation (46 ; 232) de la position d'application à la position de fixation, lequel est associé à l'un desdits au moins deux organes de fixation (48 ; 50 ; 234 ; 316 ; 329).

5. Système de maintien selon l'une des revendications 2 à 4, **caractérisé en ce qu'**une distance entre lesdits au moins deux organes de fixation (48 ; 50 ; 234 ; 316 ; 329) est, dans la position d'application, plus grande ou plus petite que dans la position de fixation.

6. Système de maintien selon l'une des revendications 2 à 5, **caractérisé en ce qu'**au moins un desdits au moins deux organes de fixation (48 ; 50 ; 234 ; 316 ; 329) comprend au moins un élément d'accouplement (62 ; 92 ; 240 ; 242), dans lequel ledit au moins un élément d'accouplement (62 ; 92 ; 240 ; 242) présente une première surface d'accouplement (64 ; 244) dirigée ou sensiblement dirigée vers le dispositif de support (20 ; 216) pour venir s'appliquer contre une surface de fixation correspondante d'un contenant chirurgical, et dans lequel le système de maintien présente au moins une deuxième surface d'accouplement (66 ; 246) dirigée à l'opposé ou sensiblement à l'opposé de la direction dirigée vers le dispositif de support (20 ; 216) pour venir s'appliquer contre au moins une surface de fixation correspondante d'un contenant chirurgical.

7. Système de maintien selon l'une des revendications précédentes, **caractérisé en ce que** le dispositif de support (20 ; 216) comprend au moins un élément de support (22 ; 218) pour maintenir et/ou supporter des instruments chirurgicaux et/ou des implants, et au moins une pièce de maintien (24 ; 220) pour maintenir ledit au moins un élément de support (22 ; 218), et **en ce que** le dispositif de fixation (46 ; 232) comprend au moins deux organes de fixation (48 ; 50 ; 234 ; 316 ; 329) reliés mutuellement par ladite au moins une pièce de maintien (24 ; 220).

8. Système de maintien selon l'une des revendications précédentes, **caractérisé en ce que** ladite au moins une pièce de maintien (24) comprend au moins un organe de maintien (34) pour maintenir ledit au moins un élément de support (22) dans la position de maintien.

9. Système de maintien selon la revendication 8, **caractérisé en ce que** ladite au moins une pièce de maintien (24) comprend au moins deux organes de maintien (34) et **en ce que** lesdits au moins deux organes de maintien (34) sont agencés de manière mobile l'un par rapport à l'autre.

10. Système de maintien selon la revendication 9, **caractérisé en ce que** lesdits au moins deux organes de maintien (34) sont agencés pivotants l'un par rapport à l'autre.

11. Système de maintien selon la revendication 9 ou 10, **caractérisé en ce que** lesdits au moins deux organes de maintien (34) présentent respectivement une première extrémité et une deuxième extrémité, et sont reliés mutuellement au niveau de leurs premières extrémités, par une liaison articulée.

12. Système de maintien selon la revendication 11, **caractérisé en ce que** la liaison articulée est réalisée sous la forme d'une charnière simple, d'une charnière à film ou d'une charnière à ressort, et/ou **en ce qu'**un organe de fixation (48) comprend la liaison articulée, et/ou **en ce qu'**au niveau de la deuxième extrémité d'au moins un organe de maintien (34) est agencé un organe de fixation (50).

13. Système de maintien selon l'une des revendications 8 à 12, **caractérisé en ce que** ledit au moins un organe de maintien (34) présente au moins un élément de maintien (116) pour maintenir par complémentarité de formes et/ou par adhérence ledit au moins un élément de support (22) dans la position de maintien.

14. Système de maintien selon l'une des revendications précédentes, **caractérisé en ce que** ledit au moins un élément de support (22) présente au moins un élément de liaison (108) qui, dans la position de maintien, est maintenu par complémentarité de formes et/ou par adhérence sur ladite au moins une pièce de maintien (24).

15. Contenant chirurgical pour stocker et/ou stériliser et/ou nettoyer des instruments chirurgicaux et/ou des implants, comprenant un compartiment d'accueil défini par un fond et des parois latérales, et au moins un système de maintien chirurgical (10 ; 210 ; 310) pour instruments chirurgicaux et/ou implants, **caractérisé en ce que** ledit au moins un système de maintien (10 ; 210 ; 310) est un système de maintien (10 ; 210 ; 310) selon l'une des revendications précédentes.
